# EUROPEAN PATENT APPLICATION

(11) **EP 4 253 434 A1**
(43) Date of publication of application: **04.10.2023**
(21) Application number: 21897113.3
(22) Date of filing: 26.11.2021
(51) Int. Cl.: C08F 220/32, C08F 220/56, C08F 220/20, C08F 222/38, C03C 17/30, B01J 20/26, B01J 20/285, C12Q 1/68, C12Q 1/37, C08F 265/06, C08F 265/10, C08F 222/14

(54) **SUBSTRATE, AND PREPARATION METHOD THEREFOR AND USE OF SUBSTRATE**

(30) Priority: 30.11.2020 CN 202011375903
(71) Applicant: GeneMind Biosciences Company Limited, Shenzhen City, Guangdong 518023 (CN)
(72) Inventor: LIN, Zhifeng, Shenzhen, Guangdong 518023 (CN); SUN, Lei, Shenzhen, Guangdong 518023 (CN); WANG, Qi, Shenzhen, Guangdong 518023 (CN); FENG, Diewen, Shenzhen, Guangdong 518023 (CN)
(74) Representative: Schiweck Weinzierl Koch Patentanwälte Partnerschaft mbB
(86) International application number: PCT/CN2021/133358
(87) International publication number: WO 2022/111613

(57) **Abstract**

The present invention relates to a substrate, and a preparation method therefor and the use thereof. What is referred to as the substrate has a surface, the surface comprising a polymer coating covalently attached thereto. The polymer coating comprises a polymer comprising a repeating unit A as represented by formula I and a repeating unit B as represented by formula II or formula III: 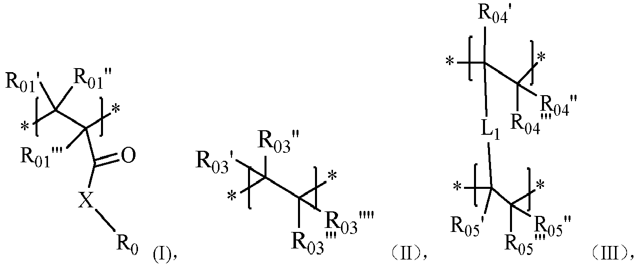 wherein X is selected from -O- or -NH-, R₀₁', R₀₁" and R₀₁‴ are each independently selected from -H or a C1-C3 alkyl, R₀ is selected from a C1-C10 alkyl or -(C1-C5 alkyl)-NH-NHS-PEG4, and R₀ comprises at least one R₀₂ substitution, at least one R₀₂ substitution each independently selected from epoxy, amino, or azido, R_{03'}, R_{03"}, R_{03‴}, R_{03‴′}, R_{04'}, R_{04"}, R_{04‴}, R_{05'}, R_{05"} and R_{05‴} are each independently selected from -H, a C1-C3 alkyl, acylamino or ester group, L₁ is selected from a C1-C3 alkylene or -C(O)-R₀₆-C(O)-, and R₀₆ is selected from PEG or alkyldiamine. The surface can load biomolecules at a higher density, can meet the evolving developments in terms of biomolecular preparation and/or analysis requirements, and has a good stability.

## Description

### TECHNICAL FIELD

The present disclosure relates to the fields of surface treatment and biomolecule detection, in particular to a substrate having a polymer coating on a surface thereof, a method for preparing the substrate having the polymer coating on the surface thereof, and use of the substrate.

### BACKGROUND

The subject matter discussed in this section should not be admitted to be prior art merely as a result of its mention in this section. A biochip/an array can be made by modifying a surface of a substrate and incorporating a polymer coating.

The surface modification technology, from two-dimensional modification to 2.5-dimensional modification and then to three-dimensional modification, can result in a significant increase in the density of functional groups contained on the surface. The increased density of functional groups makes the biochip suitable for high-throughput testing, such as hybridization, amplification, and sequencing on the chip surface.

How to treat the surface to obtain the desired parameters for the surface or to make the surface meet the requirements of application to detection, and to make the surface properties stable, controllable and easily reproducible, is a matter of concern.

### SUMMARY

The present disclosure aims to solve at least one of the above technical problems to at least some extent or to provide an alternative technical solution.

To this end, the embodiments of the present disclosure provide a substrate having a surface. The surface contains a polymer coating covalently linked thereto. The polymer coating contains a polymer including a repeating unit A of formula I and a repeating unit B of formula II or formula III: 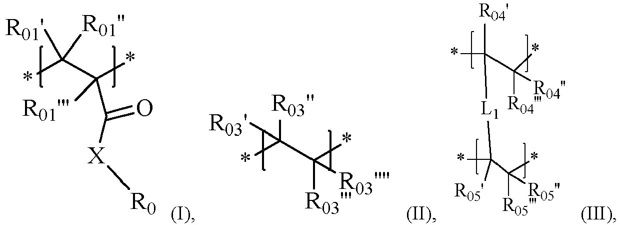 wherein,
X is selected from -O- and -NH-;
R₀₁, R₀₁", and R₀₁‴ are each independently selected from -H and C1-C3 alkyl;
R₀ is selected from C1-C10 alkyl and -(C1-C5 alkyl)-NH-NHS-PEG4, and R₀ contains at least one R₀₂ substituent, wherein at least one R₀₂ substituent is each independently selected from epoxy, amino, and azido;
R₀₃', R₀₃", R₀₃‴, R₀₃‴′, R₀₄', R₀₄", R₀₄‴, R₀₅', R₀₅", and R₀₅‴ are each independently selected from -H, C1-C3 alkyl, amido, and an ester group;
L₁ is selected from C1-C3 alkylene and -C(O)-R₀₆-C(O)-; and
R₀₆ is selected from PEG and alkyl diamine, and in the case that R₀₆ is selected from PEG, the PEG has a molecular weight of 200-2000.

The surface with the polymer coating has functional groups with high density and uniform distribution, and with high reactivity, and features stable properties and controllable reactions, such that it can meet the requirements of application scenarios of high-throughput detection on biomolecules by loading a large number of biomolecules.

The embodiments of the present disclosure further provide a method for preparing a substrate having a polymer coating on a surface thereof, which includes: making a polymer in contact with the surface to allow the polymer to be linked to the surface. The polymer contains a repeating unit A of formula I and a repeating unit B of formula II or formula III: 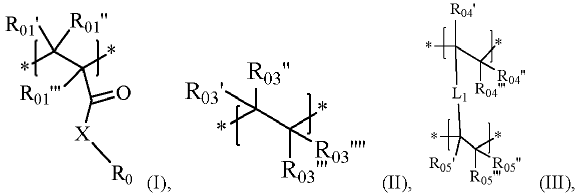 wherein,
X is selected from -O- and -NH-;
R₀₁, R₀₁", and R₀₁‴ are each independently selected from -H and C1-C3 alkyl;
R₀ is selected from C1-C10 alkyl and -(C1-C5 alkyl)-NH-NHS-PEG4, and R₀ contains at least one R₀₂ substituent, wherein at least one R₀₂ substituent is each independently selected from epoxy, amino, and azido, and the PEG4 here indicates that n in HO(CH₂CH₂O)nH is 4;
R₀₃', R₀₃", R₀₃‴, R₀₃‴′, R₀₄', R₀₄", R₀₄‴_{,} R₀₅', R₀₅", and R₀₅‴ are each independently selected from -H, C1-C3 alkyl, amido, and an ester group;
L₁ is selected from C1-C3 alkylene and -C(O)-R₀₆-C(O)-; and
R₀₆ is selected from PEG and alkyl diamine, and in the case that R₀₆ is selected from PEG, the PEG has a molecular weight of 200-2000.

The embodiments of the present disclosure further provide use of the substrate according to any one of the above embodiments or the substrate prepared by the method according to any one of the above embodiments in biomolecule capture and/or detection.

In the surface containing a polymer coating provided by the above embodiments, the polymer consists of a specific repeating unit A and a repeating unit B. The surface having the polymer coating is characterized by high density, uniform distribution, and high reactivity of functional groups, such that the surface can be loaded with biological components/biomolecules at a high density, and the evolving requirements of biomolecule preparation and/or analysis are met. In addition, the polymer on the surface has good stability and is relatively insensitive to air, such that the substrate production or surface treatment is easy to carry out, and the substrate has strong industrial practicability.

The chip according to any one of the above embodiments or the chip prepared by the method according to any one of the above embodiments has a uniformly modified surface with high biochemical activity, which is beneficial for controlling the amount and/or density of the subsequently loaded oligonucleotide sequence (primer or probe) and/or nucleic acid under test molecule directly or indirectly linked to the polymer. The chip is particularly suitable for application scenarios with high throughput requirements and the need for stable and controllable surface properties.

The substrate with the above surface properties is suitable for a sequencing platform for realizing sequencing based on chip detection and by using the sequencing by synthesis (SBS) principle, specifically, for example, a single-molecule sequencing platform that allows a nucleic acid molecule under test to be linked to the surface of the chip for single-molecule detection directly without amplification, or for example, a high-throughput sequencing platform that allows a nucleic acid molecule under test to be linked to the surface of the chip and then detected after being amplified into clusters (amplifying signals) on the surface. The substrate/chip is suitable for a mainstream platform for realizing sequencing based on the SBS principle currently on the market, such as sequencing platforms of ILLUMINA, BGI, and the like. In addition, by using the method according to the above embodiments, the chips with stable and consistent surface properties can be easily and controllably prepared in batches, and have strong industrial practicability.

### BRIEF DESCRIPTION OF THE DRAWINGS

The above and/or additional aspects and advantages of the embodiments of the present disclosure will become apparent and easily understood from the description of the embodiments in reference to the following drawings, among which:
FIG. 1 is a schematic diagram of a process for preparing a chip according to an embodiment of the present disclosure;
FIG. 2 is a diagram showing the molecular weight of the polymer prepared according to Example 1 of the present disclosure by a GPC test;
FIG. 3 is a light intensity image for quality test of the surface of the chip prepared according to Example 1 of the present disclosure;
FIG. 4 is a light intensity image for biological applications of the chip prepared according to Example 1 of the present disclosure;
FIG. 5 is a diagram showing the molecular weight of the polymer prepared according to Example 2 of the present disclosure by a GPC test;
FIG. 6 is a light intensity image for quality test of the surface of the chip prepared according to Example 2 of the present disclosure;
FIG. 7 is a light intensity image for biological applications of the chip prepared according to Example 2 of the present disclosure;
FIG. 8 is a diagram showing the molecular weight of the polymer prepared according to Example 3 of the present disclosure by a GPC test;
FIG. 9 is a schematic diagram of nuclear magnetic resonance detection of the azido macromolecule in Example 3 of the present disclosure;
FIG. 10 is a light intensity image for quality test of the surface of the chip prepared according to Example 3 of the present disclosure;
FIG. 11 is a light intensity diagram for biological applications of the chip prepared according to Example 3 of the present disclosure;
FIG. 12 is a diagram showing the molecular weight of the polymer prepared according to Example 4 of the present disclosure by a GPC test;
FIG. 13 is a light intensity image for quality test of the surface of the chip prepared according to Example 4 of the present disclosure;
FIG. 14 is a light intensity image for biological applications of the chip prepared according to Example 4 of the present disclosure;
FIG. 15 is a diagram showing the molecular weight of the polymer prepared according to Example 5 of the present disclosure by a GPC test;
FIG. 16 is a light intensity image for quality test of the surface of the chip prepared according to Example 5 of the present disclosure;
FIG. 17 is a light intensity image for biological applications of the chip prepared according to Example 5 of the present disclosure;
FIG. 18 is a diagram showing the molecular structure of the library in Example 6 of the present disclosure; and
FIGs. 19-23 are fluorescence intensity detection images of the chips prepared in Examples 1-5 of the present disclosure, wherein the left image is an original image, and the right image is an image processed by ImageJ binarization.

### DETAILED DESCRIPTION

The substrate/chip of the present disclosure, the method for preparing the same, and the use of the same are described in further details below with reference to specific examples. The embodiments described below with reference to the drawings are exemplary and are merely intended to explain the present disclosure rather than be construed as limiting the present disclosure.

Unless otherwise defined, all technical and scientific terms used herein have the same meaning as commonly understood by those skilled in the art to which the present disclosure belongs. Unless otherwise specified, reagents, detection instruments, etc., in the examples can be self-prepared or are commercially available. As used herein, the term "and/or" includes any and all combinations of one or more of the associated listed items. In addition, since most of the specific data referred to herein are statistically significant or difficult to determine precisely, unless otherwise specified, any numerical value expressed in a precise manner is meant to be a range, e.g., an interval containing plus or minus 10% of the numerical value, which will not be repeated herein.

The chip used herein includes a substrate having a polymer coating on a surface thereof, and also includes a substrate having a polymer coating linked with a biomolecule. The material of the substrate is not particularly limited, and is, for example, at least one selected from glass, silicon wafer, plastic, gel, and nylon film. Unless otherwise specified, the substrate, chip, and biochip are used interchangeably.

"Linking" as used herein should be comprehended in its broad sense. For example, it may be direct connection or indirect connection through an intermediate, and it may be chemical ligation or physical connection. Unless otherwise specifically defined, in the description herein of a connection relationship involving compounds, biomolecules, functional groups, groups, etc., "linking" generally refers to chemical ligation, such as binding through a covalent bond, adsorption on the basis of Van der Waals' force or electrostatic interaction, or the like. For those skilled in the art, the specific meanings of the above terms herein can be understood according to specific conditions.

The polymer as used herein refers to a polymer compound generally having a molecular weight greater than 1000, which is sometimes referred to as a high polymer. The polymer herein is a polymer formed by a plurality of monomer molecules undergoing polymerization, e.g., addition polymerization.

The functional group and active group as referred to herein are used interchangeably to refer to groups that impart a property or properties to a compound.

"Grafted to... via..." or "modified with..." as used herein may refer to direct grafting to or modification with an object, or may refer to indirect grafting to or modification with the object, for example, via other transition groups or structures. Unless otherwise specifically stated, the linking herein includes grafting, immobilization, binding, and the like; also, grafting, immobilization, binding and covalent linking/binding (linking via a covalent bond) are used interchangeably herein.

The term "alkyl" refers to a saturated hydrocarbon containing a primary (normal) carbon atom, a secondary carbon atom, a tertiary carbon atom, a quaternary carbon atom, or a combination thereof. For example, "C₁-C₁₀ alkyl" refers to alkyl groups containing 1-10 carbon atoms, which may be independently C₁ alkyl, C₂ alkyl, C₃ alkyl, C₄ alkyl, C₅ alkyl, C₆ alkyl, C₇ alkyl, C₈ alkyl, C₉ alkyl, or C₁₀ alkyl at each occurrence. The alkyl groups include, but are not limited to: methyl (Me, -CH₃), ethyl (Et, -CH₂CH₃), 1-propyl (n-Pr, n-propyl, -CH₂CH₂CH₃), 2-propyl (i-Pr, i-propyl, -CH(CH₃)₂), 1-butyl (n-Bu, n-butyl, -CH₂CH₂CH₂CH₃), 2-methyl-1-propyl (i-Bu, i-butyl, -CH₂CH(CH₃)₂), 2-butyl (s-Bu, s-butyl, -CH(CH₃)CH₂CH₃), 2-methyl-2-propyl (t-Bu, t-butyl, -C(CH₃)₃), 1-pentyl (n-pentyl, -CH₂CH₂CH₂CH₂CH₃), 2-pentyl (-CH(CH₃)CH₂CH₂CH₃), 3-pentyl (-CH(CH₂CH₃)₂), 2-methyl-2-butyl (-C(CH₃)₂CH₂CH₃), 3-methyl-2-butyl (-CH(CH₃)CH(CH₃)₂), 3-methyl-1-butyl (-CH₂CH₂CH(CH₃)₂), 2-methyl-1-butyl(-CH₂CH(CH₃)CH₂CH₃), 1-hexyl (-CH₂CH₂CH₂CH₂CH₂CH₃), 2-hexyl (-CH(CH₃)CH₂CH₂CH₂CH₃), 3-hexyl (-CH(CH₂CH₃)(CH₂CH₂CH₃)), 2-methyl-2-pentyl (-C(CH₃)₂CH₂CH₂CH₃), 3-methyl-2-pentyl (-CH(CH₃)CH(CH₃)CH₂CH₃), 4-methyl-2-pentyl (-CH(CH₃)CH₂CH(CH₃)₂), 3-methyl-3-pentyl (-C(CH₃)(CH₂CH₃)₂), 2-methyl-3-pentyl (-CH(CH₂CH₃)CH(CH₃)₂), 2,3-dimethyl-2-butyl (-C(CH₃)₂CH(CH₃)₂), 3,3-dimethyl-2-butyl (-CH(CH₃)C(CH₃)₃, and octyl (-(CH₂)₇CH₃).

"Epoxy" refers to groups containing the structure -X-CH(O)CH-X, wherein each X is independently H or alkyl. The epoxy groups include, but are not limited to, -CH(O)CH₂ and -CH(O)CH(alkyl).

"Amino" refers to derivatives of ammonia that are groups containing the structure -N(X)₂, wherein each X is independently H or alkyl. The amino groups include, but are not limited to, -NH₂, -N(alkyl)₂, -NH(alkyl), -N(cycloalkyl)₂, -NH(cycloalkyl), -N(heterocyclyl)₂, -NH(heterocyclyl), -N(aryl)₂, -NH(aryl), -N(alkyl)(aryl), -N(alkyl)(heterocyclyl), -N(cycloalkyl)(heterocyclyl), -N(aryl)(heteroaryl), and -N (alkyl)(heteroaryl).

"Azido" or "azide" refers to -N₃.

"Ester group" refers to groups containing the structure -C(O)O-X, wherein X is alkyl. The ester groups include, but are not limited to, -C(O)OCH₃ and -C(O)OCH₂CH₃.

"Amido" refers to groups containing the structure -C(O)N(X)₂, wherein each X is independently H or alkyl. The amide groups include, but are not limited to, -C(O)NH₂, -C(O)NH(alkyl), and -C(O)N(alkyl)₂.

The biomolecule or biological component as used herein includes a nucleic acid and/or a protein. The nucleic acid may be DNA, cDNA, RNA or an RNA-DNA complex, and may be double-stranded or single-stranded.

The probe/primer as used herein is a nucleic acid molecule of known sequence that can be used to capture a target nucleic acid sequence or as a primer for amplification of the target nucleic acid sequence. It may be DNA and/or RNA, etc., and is generally an oligonucleotide strand with a length of less than 150 nt. By structurally and/or chemically treating the surface of the substrate, the probes linked to the surface can be made to be randomly or regularly distributed.

The specific embodiments of the present disclosure provide a substrate having a surface. The surface contains a polymer coating covalently linked thereto. The polymer coating contains a polymer including a repeating unit A of formula I and a repeating unit B of formula II or formula III: 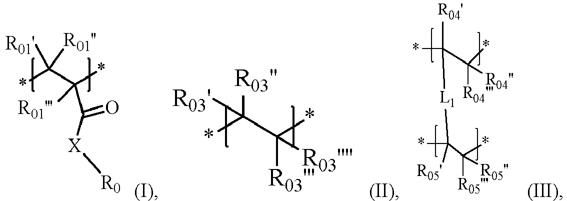 wherein, X is selected from -O- and -NH-; R₀₁', R₀₁", and R₀₁‴ are each independently selected from -H and C1-C3 alkyl; R₀ is selected from C1-C10 alkyl and -(C1-C5 alkyl)-NH-NHS-PEG4, and R₀ contains at least one R₀₂ substituent, wherein at least one R₀₂ substituent is independently selected from epoxy, amino, and azido, and the PEG4 here indicates that n in HO(CH₂CH₂O)nH is 4; R₀₃', R₀₃", R₀₃‴, R₀₃‴′ R₀₄', R₀₄', R₀₄‴, R₀₅', R₀₅", and R₀₅‴ are each independently selected from -H, C1-C3 alkyl, amido, and an ester group; L₁ is selected from C1-C3 alkylene and -C(O)-R₀₆-C(O)-; and R₀₆ is selected from PEG and alkyl diamine.

In a certain example, R₀₆ is selected from PEG, which has a molecular weight of 200-2000, preferably 500-1000.

In certain examples, the polymer contains a structure of formula III, formula IV, or formula V:

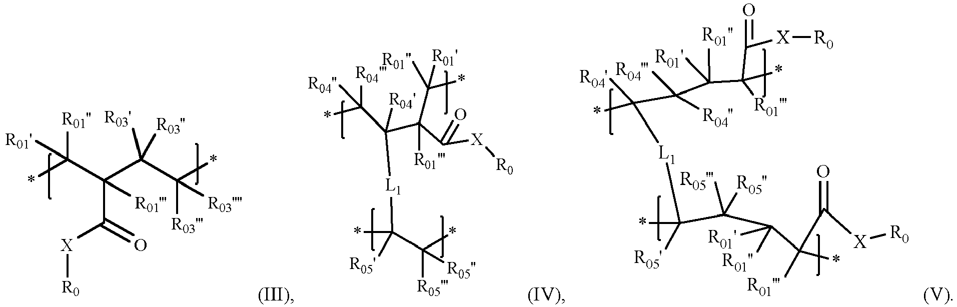

In certain examples, the polymer coating contains a polymer of formula VI or formula VII: 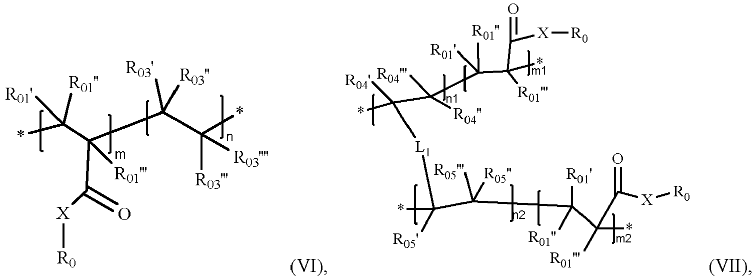 wherein, m is selected from integers in the range of 1-2000, n is selected from integers in a range of 1-3000, m1 and m2 are each independently selected from integers in a range of 1-2000, and n1 and n2 are selected from integers in a range of 1-1500 and n1 is equal to n2, and in the case that R₀₆ is selected from PEG, the PEG has a molecular weight of less than 1000, preferably 500-1000.

In certain examples, the polymer coating contains the polymer of formula VI, wherein a ratio of m to n is 1:1-1:30, preferably 1:2-1:30, and more preferably 1:10-1:20.

In certain examples, the polymer coating contains the polymer of formula VII, wherein a ratio of the sum of m1 and m2 to n1 or n2 is 1:1-1:30, preferably 1:2-1:30, and more preferably, 1:10-1:20.

In certain examples, the polymer has a molecular weight of 1-200,000. The linking of polymers having molecular weights in this range to the surface can provide the surface with desired and stable and controllable properties. Regarding the effect of polymer molecular weight on surface properties, for high polymers in this molecular weight range formed by polymerization in the same monomer ratio, based on the knowledge of high polymers and the morphology/structure of the high polymers on the surface and from test experience, the inventor speculates that a polymer with a relatively high molecular weight, e.g., 180,000, will result in the surface with superior properties, e.g., a larger number of functional groups, a more uniform distribution, etc., compared with a polymer with a relatively low molecular weight, e.g., 20,000. However, surprisingly, from the test in which two surfaces having polymer coatings of these two molecular weights, respectively, are linked to the same probe, it is found that the properties of the surfaces having polymer coatings of these two molecular weights are comparable, with no significant difference.

In certain examples, the polymer coating is linked to the surface by covalently binding to an active group on the surface. The active group is selected from at least one of amino, epoxy, alkynyl, cyano, vinyl, and propenyl.

In certain examples, the active group is an amino group, and the polymer contains an epoxy group. At least a portion of the amino group is subjected to a reaction with at least a portion of the epoxy group on the polymer to allow the polymer to be covalently bound to the surface through the active group.

In certain examples, the active group is an epoxy group and the polymer contains -NH₂. At least a portion of the epoxy group is subjected to a reaction with at least a portion of -NH₂ on the polymer to allow the polymer to be covalently bound to the surface through the active group.

In certain examples, the active group is one of alkynyl, cyano, vinyl, and propenyl, and the polymer contains -N₃. At least a portion of the designated active group is subjected to a reaction with at least a portion of N₃ on the polymer to allow the polymer to be covalently bound to the surface through the active group.

In certain examples, X is selected from -O-.

Specifically, in some examples, R₀₁', R₀₁", and R₀₁‴ are each independently selected from -H and methyl, and R₀ is selected from C1-C3 alkyl; and/or R₀₃', R₀₃", R₀₃‴, R₀₃‴′ R₀₄', R₀₄", R₀₄‴, R₀₅', R₀₅", and R₀₅‴ are each independently selected from -H, methyl, -C(O)NH₂, and -C(O)OCH₃, and L₁ is selected from -C(O)-PEG-C(O)- and -C(O)-NH-CH₂-NH-C(O)-.

In certain examples, the repeating unit A is selected from one of the following structures:

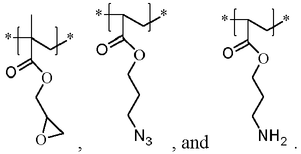

In some examples, the repeating unit B is selected from one of the following structures: 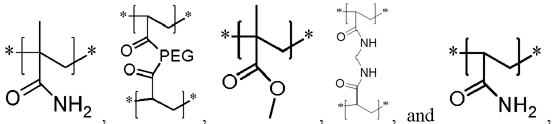 wherein the PEG has a molecular weight of 200-2000, preferably 500-1000.

In some examples, the polymer contains one of the following structures:

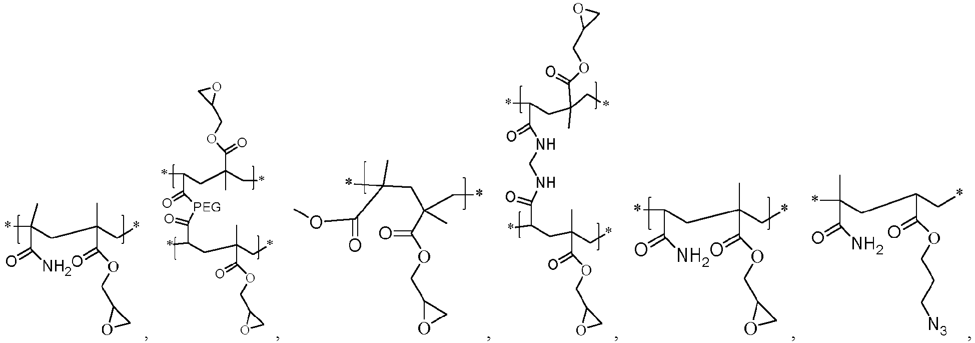

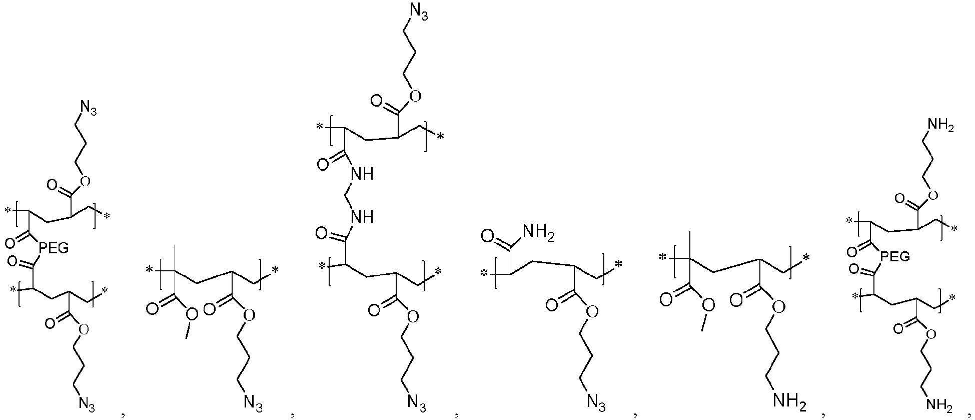

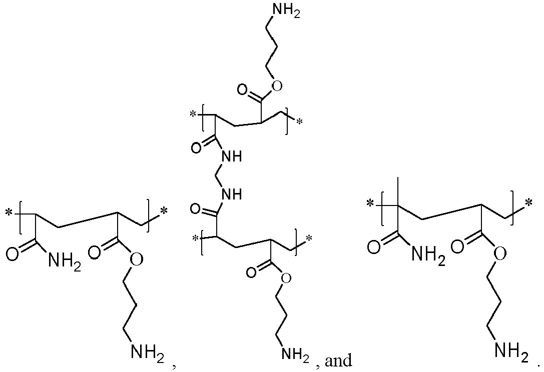

In some examples, the polymer coating contains one of the following polymers: 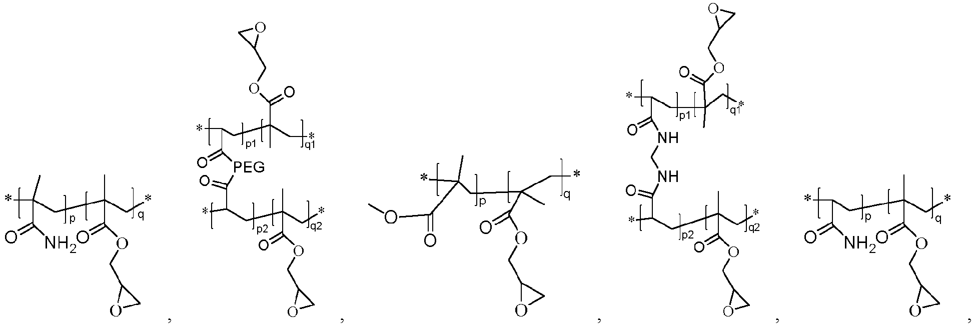 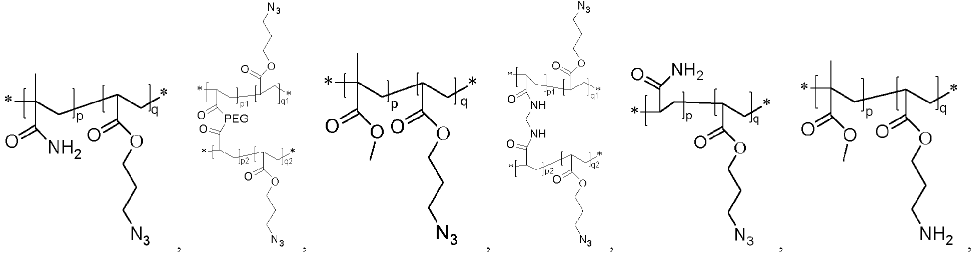 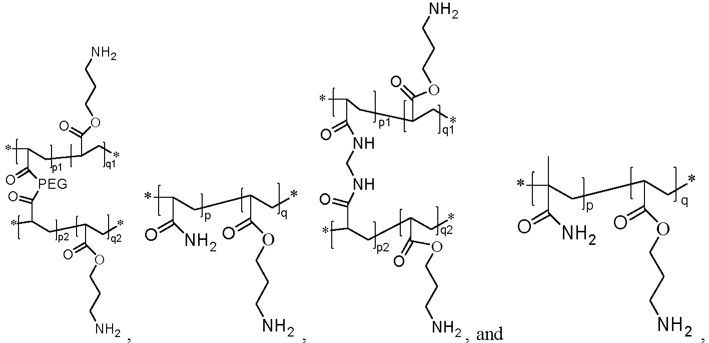 wherein p is selected from integers in a range of 1-3000, p1 and p2 are each independently selected from integers in a range of 1-1300 and p1 is equal to p2, and q, q1, and q2 are each independently selected from integers in a range of 1-2000.

In certain other examples, X is selected from -NH-.

Specifically, in certain examples, the repeating unit B is as shown in formula III.

In some examples, R₀₁', R₀₁", and R₀₁‴ are each independently selected from -H and methyl, and R₀ is selected from C1-C3 alkyl and -(C1-C5 alkyl)-NH-NHS-PEG4; and/or R₀₄', R₀₄", R₀₄‴, R₀₅', R₀₅", and R₀₅‴ are each independently selected from -H, methyl, -C(O)NH₂ or -C(O)OCH₃, and L₁ is selected from -C(O)-PEG-C(O)- and -C(O)-NH-CH₂-NH-C(O)-, in the case that L₁ is selected from -C(O)-PEG-C(O)-, the PEG has a molecular weight of 200-2000, preferably 500-1000.

In certain examples, the repeating unit A is selected from one of the following structures:

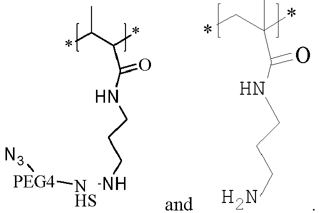

The PEG4 here indicates HO(CH₂CH₂O)nH, wherein n in HO(CH₂CH₂O)nH is 4.

In some examples, the repeating unit B is selected from one of the following structures:

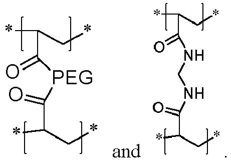

In some examples, the polymer contains one of the following structures:

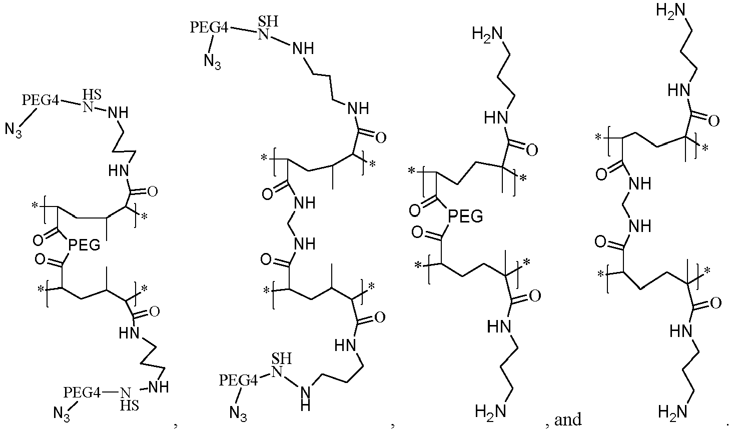

In some examples, the polymer coating contains one of the following polymers: 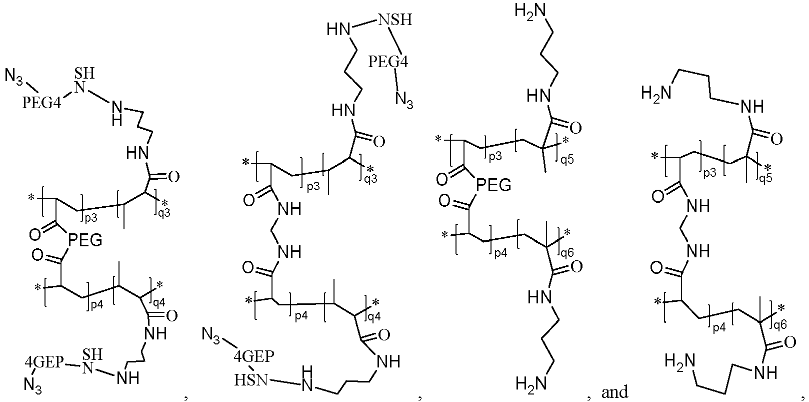 wherein, p3 and p4 are each independently selected from integers in a range of 1-1300 and p3 is equal to p4, q3 and q4 are each independently selected from integers in a range of 1-500, and q5 and q6 are each independently selected from integers in a range of 1-1500.

In some other examples, the repeating unit B is as shown in formula II.

Specifically, in some examples, the repeating unit B is selected from one of the following structures: 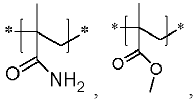 and

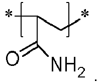

In some examples, R₀ is selected from -(C1-C5 alkyl)-NH-NHS-PEG4-N₃. The PEG4 here indicates HO(CH₂CH₂O)nH, wherein, that n in HO(CH₂CH₂O)nH is 4.

In some examples, the repeating unit A is

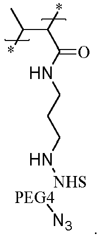

In some examples, the polymer includes one of the following structures: 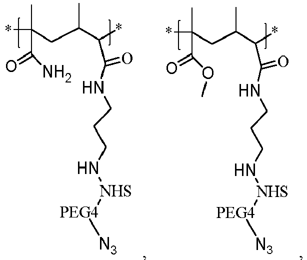 and

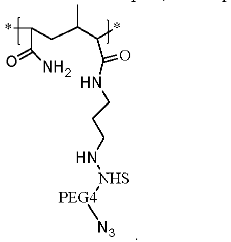

In some examples, the polymer coating includes one of the following polymers: 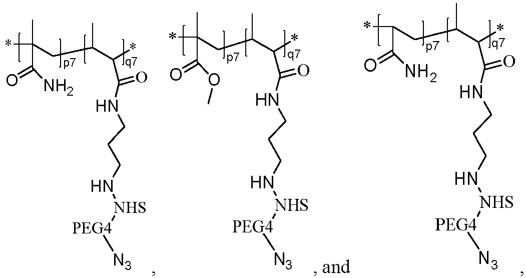 wherein, p7 is selected from integers in a range of 1-3000, and q7 is selected from integers in a range of 1-500.

In certain examples, a monomer A of formula VIII corresponding to the repeating unit A and a monomer B of formula IX or formula X corresponding to the repeating unit B are subjected to a polymerization reaction to obtain the polymer, wherein the polymerization reaction is performed in the presence of an initiator, 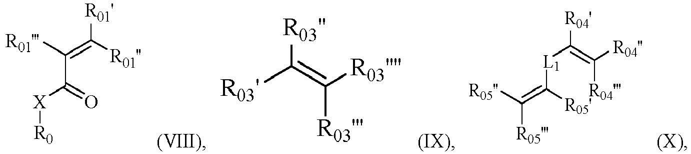 wherein,
X is selected from -O- and -NH-; R₀₁', R₀₁", and R₀₁‴ are each independently selected from -H and C1-C3 alkyl; R₀ is selected from C1-C10 alkyl and -(C1-C5 alkyl)-NH-NHS-PEG4, and R₀ contains at least one R₀₂ substituent, wherein at least one R₀₂ substituent is independently selected from epoxy, amino, and azido, and the PEG4 here indicates HO(CH₂CH₂O)nH, wherein, n in HO(CH₂CH₂O)nH is 4; R₀₃', R₀₃", R₀₃‴, R₀₃‴′, R₀₄', R₀₄", R₀₄‴, R₀₅', R₀₅", and R₀₅‴ are each independently selected from -H, C1-C3 alkyl, amido, and an ester group; L₁ is selected from C1-C3 alkylene and -C(O)-R₀₆-C(O)-; and R₀₆ is selected from PEG and alkyl diamine.

In certain specific examples, in the case that R₀₆ is PEG, the PEG has a molecular weight of 200-2000, preferably 500-1000.

In certain examples, the molar ratio of the monomer A and the monomer B in the polymerization reaction is selected from a numerical value in a range of 1:1-1:30, preferably a numerical value in a range of 1:2-1:30, and more preferably a numerical value in a range of 1: 10-1 :20.

In certain examples, the surface is further linked with a biomolecule, which is linked to the surface by covalently binding to the polymer.

In certain examples, the biomolecule is selected from at least one of a protein and a nucleic acid. In a certain example, the biomolecule is, for example, a probe that has a terminus with a group capable of binding to a polymer so as to be immobilized to a surface ; further, the probe may hybridize to a target nucleic acid molecule, and the biomolecule may further include a target nucleic acid molecule or a probe-target nucleic acid molecule complex.

Another specific embodiment of the present disclosure provides a method for preparing a substrate having a polymer coating on a surface thereof. The method may be used for the preparation of the substrate according to any one of the above embodiments or examples. The method includes: making a polymer in contact with the surface to allow the polymer to be linked to the surface. The polymer contains a repeating unit A of formula I and a repeating unit B of formula II or formula III: 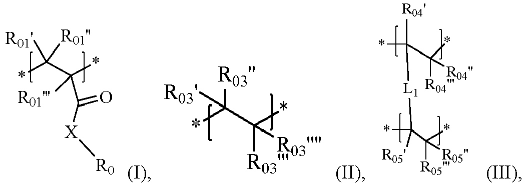 wherein, X is selected from -O- and -NH-; R₀₁', R₀₁", and R₀₁‴ are each independently selected from -H and C1-C3 alkyl; R₀ is selected from C1-C10 alkyl and -(C1-C5 alkyl)-NH-NHS-PEG4, and R₀ contains at least one R₀₂ substituent, wherein at least one R₀₂ substituent is independently selected from epoxy, amino, and azido; R₀₃', R₀₃", R₀₃‴, R₀₃‴′, R₀₄', R₀₄", R₀₄‴, R₀₅', R₀₅", and R₀₅‴ are each independently selected from -H, C1-C3 alkyl, amido, and an ester group; L₁ is selected from C1-C3 alkylene and -C(O)-R₀₆-C(O)-; and R₀₆ is selected from PEG and alkyl diamine.

It will be understood that the substrate according to any one of the above examples has technical features and advantages, and is equally suitable for the method according to this embodiment.

For example, in certain examples, the polymer contains a structure of formula III, formula IV, or formula V:

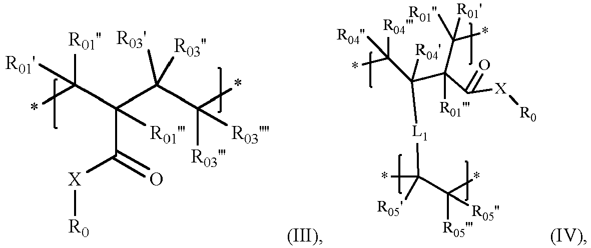

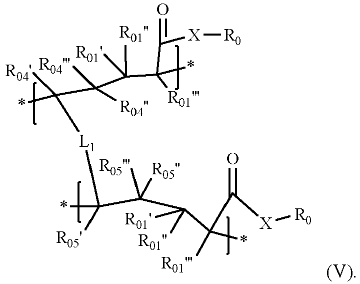

In certain examples, the polymer coating contains a polymer of formula VI or formula VII: 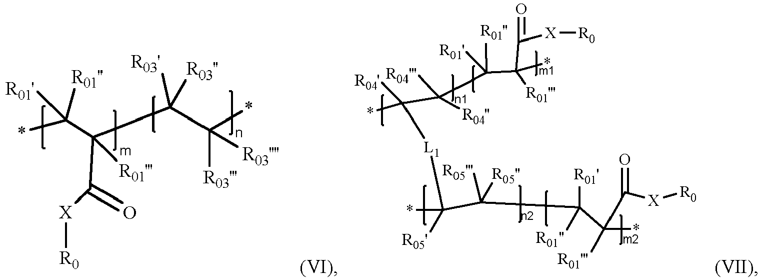 wherein, m is selected from integers in a range of 1-2000, n is selected from integers in a range of 1-3000, m1 and m2 are each independently selected from integers in a range of 1-2000, and n1 and n2 are selected from integers in a range of 1-1500 and n1 is equal to n2, and in the case that R₀₆ is selected from PEG, the PEG has a molecular weight of 200-2000, preferably 500-1000.

In certain examples, the polymer coating contains the polymer of formula VI, wherein a ratio of m to n is 1:1-1:30, preferably 1:2-1:30, and more preferably 1:10-1:20.

In certain examples, the polymer coating contains the polymer of formula VII, wherein a ratio of the sum of m1 and m2 to n1 or n2 is 1:1-1:30, preferably 1:2-1:30, and more preferably, 1:10-1:20.

In certain examples, the polymer has a molecular weight of 1-200,000.

Specifically, in some examples, X is selected from -O-.

In some examples, R₀₁', R₀₁", and R₀₁‴ are each independently selected from -H and methyl, and R₀ is selected from C1-C3 alkyl; and/or R₀₃', R₀₃", R₀₃‴, R₀₃‴′ R₀₄', R₀₄", R₀₄‴, R₀₅', R₀₅", and R₀₅‴ are each independently selected from -H, methyl, -C(O)NH₂, and -C(O)OCH₃, and L₁ is selected from -C(O)-PEG-C(O)- and -C(O)-NH-CH₂-NH-C(O)-.

In some examples, the repeating unit A is selected from one of the following structures:

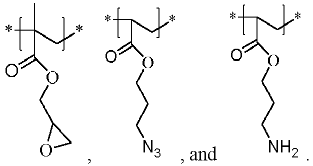

In some examples, the repeating unit B is selected from one of the following structures: 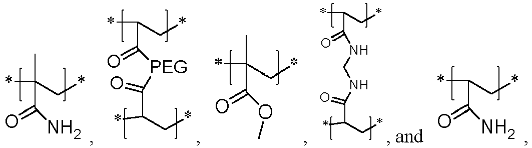 wherein the PEG has a molecular weight of 200-2000, e.g., no more than 1000, preferably 500-1000.

In some examples, the polymer contains one of the following structures:

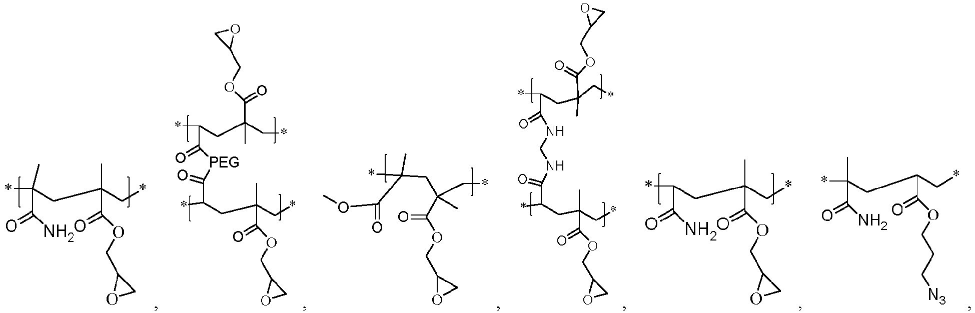

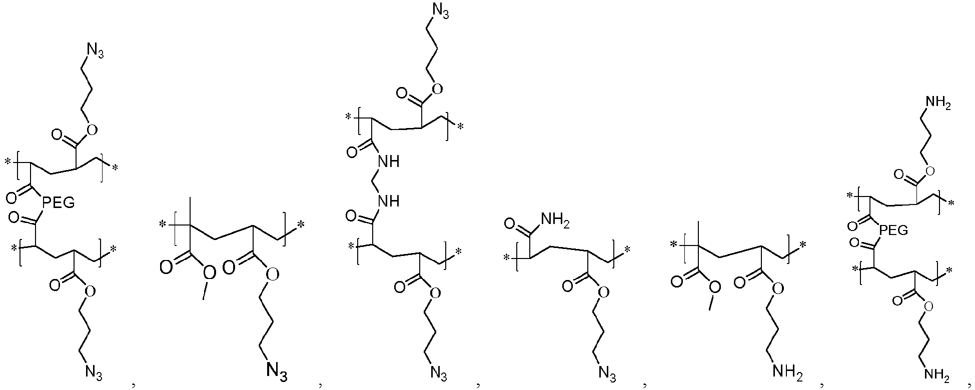

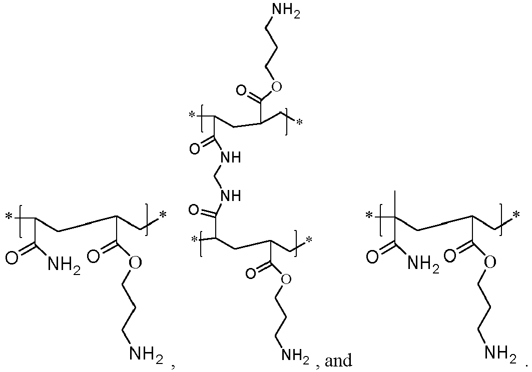

In some examples, the polymer contains one of the following polymers: 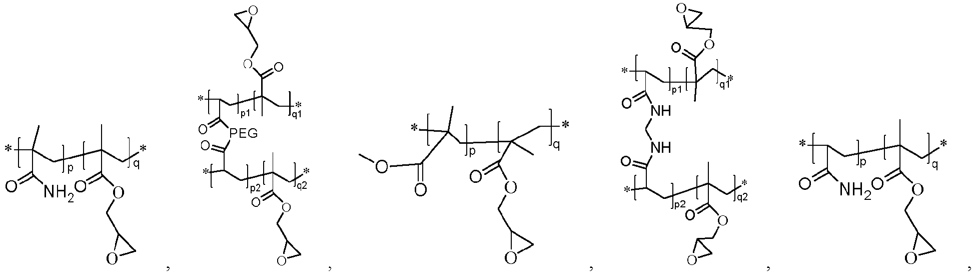 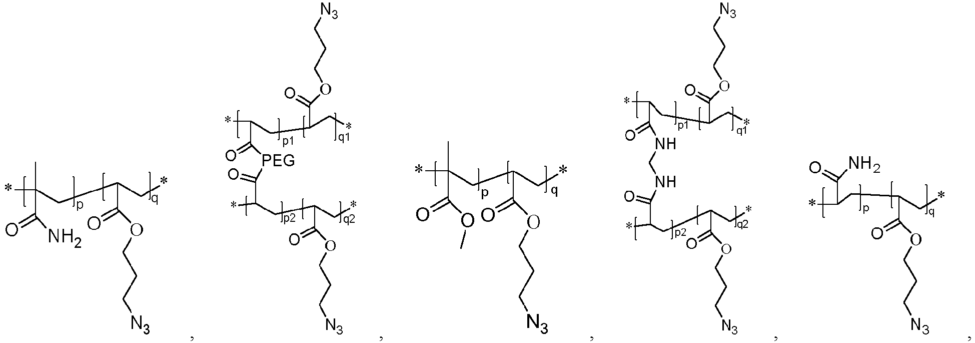 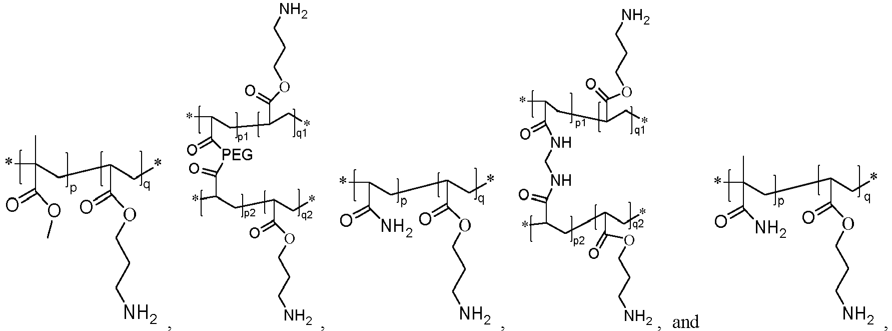 wherein p is selected from integers in a range of 1-3000, p1 and p2 are each independently selected from integers in a range of 1-1300 and p1 is equal to p2, and q, q1, and q2 are each independently selected from integers in a range of 1-2000.

In some other examples, X is selected from -NH-. Specifically, the repeating unit B is as shown in formula III.

More specifically, in certain examples, R₀₁', R₀₁", and R₀₁‴ are each independently selected from -H and methyl, and R₀ is selected from C1-C3 alkyl and -(C1-C5 alkyl)-NH-NHS-PEG4; and/or R₀₄', R₀₄", R₀₄‴, R₀₅', R₀₅", and R₀₅‴ are each independently selected from -H, methyl, -C(O)NH₂ and -C(O)OCH₃, and L₁ is selected from -C(O)-PEG-C(O)- and -C(O)-NH-CH₂-NH-C(O)-.

In certain examples, the repeating unit A is selected from one of the following structures:

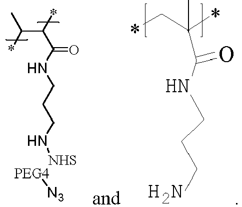

In certain examples, the repeating unit B is selected from one of the following structures:

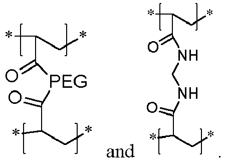

In certain examples, the polymer contains one of the following structures:

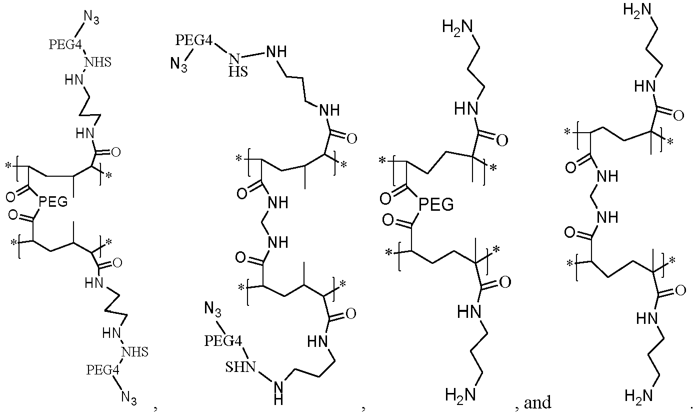

In certain examples, the polymer contains one of the following polymers: 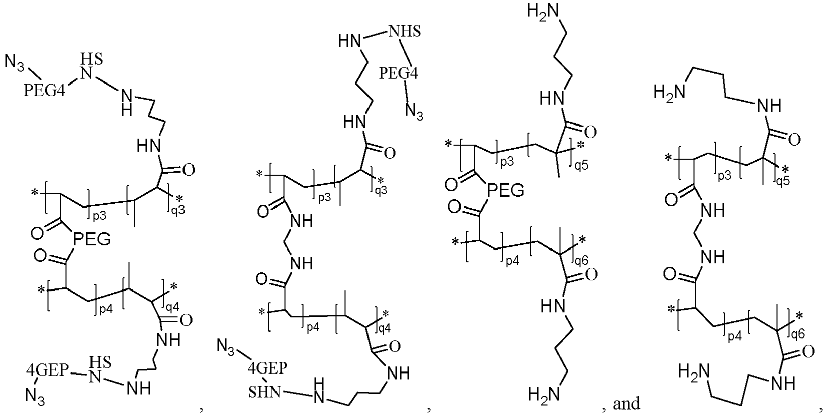 wherein, p3 and p4 are each independently selected from integers in a range of 1-1300 and p3 is equal to p4, q3 and q4 are each independently selected from integers in a range of 1-500, and q5 and q6 are each independently selected from integers in a range of 1-1500.

Specifically, in some other examples, the repeating unit B is as shown in formula II.

For example, the repeating unit B is selected from one of the following structures:

In certain examples, R₀ is selected from -(C1-C5 alkyl)-NH-NHS-PEG4-N₃.

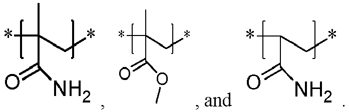

In certain examples, the repeating unit A is

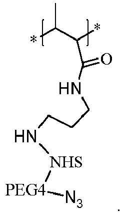

In certain examples, the polymer includes one of the following structures:

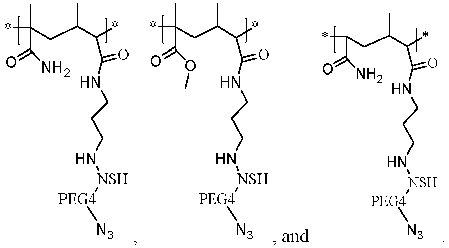

In certain examples, the polymer includes one of the following polymers: 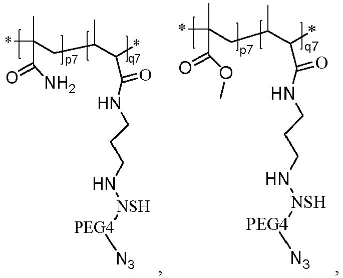 and 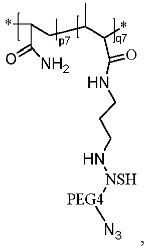 wherein, p7 is selected from integers in a range of 1-3000, and q7 is selected from integers in a range of 1-500.

In some other examples, the method includes: modifying the surface to provide the surface with an active group selected from at least one of amino, epoxy, alkynyl, cyano, vinyl, and propenyl; and making a polymer in contact with the surface to allow the polymer to be linked to the surface by covalently binding to the polymer.

Specifically, in one example, the active group is an amino group and the polymer contains an epoxy group. At least a portion of the epoxy group is subjected to a reaction with at least a portion of the amino group to allow the polymer to be linked to the surface.

In another example, the active group is an epoxy group, and the polymer contains an amino group. At least a portion of the amino group is subjected to a reaction with at least a portion of the epoxy group to allow the polymer to be linked to the surface.

In still other examples, the active group is selected from one of alkynyl, cyano, vinyl, and propenyl, and the polymer contains -N₃. At least a portion of the -N₃ is subjected to a reaction with at least a portion of the active group to allow the polymer to be linked to the surface.

In certain examples, the surface is treated with a silane coupling agent to provide the surface with the active group. The silane coupling agent is selected from at least one of 3-aminopropyltrimethoxysilane, 2-propynyl[3-(triethoxysilyl)propyl]carbamate, 4-(triethoxy)silylbutyronitrile, and γ-(2,3-epoxypropoxy)propyltrimethoxysilane.

In certain examples, a reaction is performed at 40-50 °C for 1-8 h to allow the polymer to be linked to the surface by covalently binding to the active group.

In certain examples, the method further includes: allowing the biomolecule to be linked to the surface by covalently binding to the polymer.

In certain examples, the biomolecule is selected from at least one of a protein and a nucleic acid.

In certain examples, a reaction is performed at 50-60 °C for 0.5-5 h to allow the biomolecule to be covalently bound to the polymer.

In certain examples, the polymer contains at least an epoxy group, and the biomolecule has an amino modification on at least one terminus.

In certain examples, the polymer contains at least an amino group, and the biomolecule has NHS on at least one terminus.

In certain examples, the polymer contains -N₃, and the biomolecule has DBCO on at least one terminus.

The specific embodiments of the present disclosure further provide use of the substrate according to any one of the above embodiments or examples in biomolecule capture and/or detection. The application or use is, for example, nucleic acid detection, and more specifically, for example, sequencing.

One embodiment of the present disclosure provides a polymer. The polymer contains a repeating unit A and a repeating unit B. The repeating unit A has the structural feature as shown below: 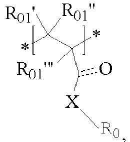 wherein X is selected from -O- and -NH- R₀₁', R₀₁", and R₀₁‴ are each independently selected from -H and C1-C3 alkyl; and R₀ is selected from C1-C10 alkyl and -(C1-C5 alkyl)-NH-NHS-PEG4, and R₀ is substituted with at least one R₀₂, wherein R₀₂ are each independently selected from epoxy, amino and azido. The repeating unit B has the structural feature as shown below: 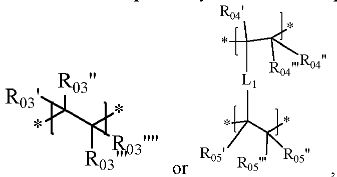 wherein R₀₃', R₀₃", R₀₃‴, R₀₃‴′, R₀₄', R₀₄", R₀₄‴, R₀₅', R₀₅", and R₀₅‴ are each independently selected from -H, C1-C3 alkyl, amido, and an ester group; L₁ is selected from C1-C3 alkylene and -C(O)-R₀₆-C(O)-; and R₀₆ is selected from PEG and alkyl diamine.

In one of the specific embodiments, X is selected from -O-.

Further, R₀₁', R₀₁", and R₀₁‴ are each independently selected from -H and methyl; and R₀ is selected from C1-C3 alkyl.

Further, R₀₃', R₀₃", R₀₃‴, R₀₃‴′, R₀₄', R₀₄", R₀₄‴, R₀₅', R₀₅", and R₀₅‴ are each independently selected from -H, methyl, -C(O)NH₂, and -C(O)OCH₃; and L₁ is selected from -C(O)-PEG-C(O)- and -C(O)-NH-CH₂-NH-C(O)-.

Specifically, the repeating unit A is selected from one of repeating units as shown below:

Specifically, the repeating unit B is selected from one of repeating units as shown below:

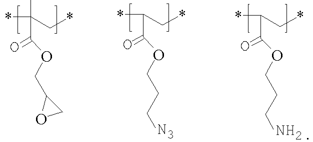

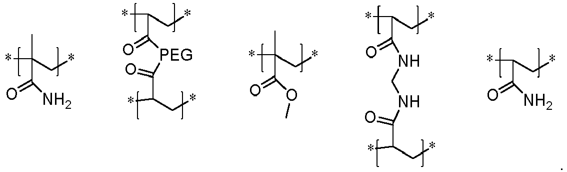

In another specific embodiment, X is selected from -NH-.

In some embodiments" the repeating unit B has the structural feature as shown below: 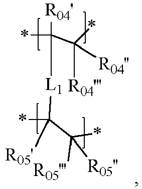 wherein R₀₄', R₀₄", R₀₄‴, R₀₅', R₀₅', and R₀₅‴ are each independently selected from -H, C1-C3 alkyl, amido, and an ester group; L₁ is selected from C1-C3 alkylene and -C(O)-R₀₆-C(O)-; and R₀₆ is selected from PEG and alkyl diamine.

Still further, R₀₁', R₀₁", and R₀₁‴ are each independently selected from -H and methyl; and R₀ is selected from C1-C3 alkyl and -(C1-C5 alkyl)-NH-NHS-PEG4.

Still further, R₀₄', R₀₄", R₀₄‴, R₀₅', R₀₅", and R₀₅‴ are each independently selected from -H, methyl, -C(O)NH₂ and -C(O)OCH₃, and L₁ is selected from -C(O)-PEG-C(O)- and -C(O)-NH-CH₂-NH-C(O)-.

Specifically, the repeating unit A is selected from one of repeating units as shown below:

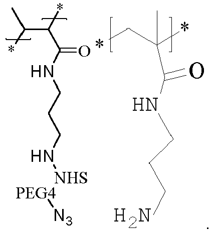

Specifically, the repeating unit B is selected from one of repeating units as shown below:

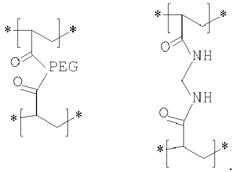

Additionally, further, the repeating unit B has the structural feature as shown below: 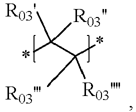 wherein R₀₃', R₀₃", R₀₃‴, and R₀₃ are each independently selected from -H, C1-C3 alkyl, amido, and an ester group.

Specifically, the repeating unit B is one of the repeating units as shown below:

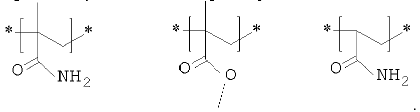

Still further, R₀ is selected from -(C1-C5 alkyl)-NH-NHS-PEG4-N₃.

Specifically, the repeating unit A is

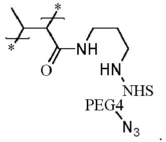

In addition, in one of the specific embodiments, the polymer has a molecular weight of 10,000-120,000.

The embodiments of the present disclosure further provide a method for preparing the above polymer, which includes: subjecting a monomer A and a monomer B to a copolymerization reaction to prepare the polymer. The monomer A forms the repeating unit A and the monomer B forms the repeating unit B.

In one of the specific embodiments, the molar ratio of the monomer A to the monomer B is 1: (1-30). Specifically, the molar ratio of the monomer A to the monomer B is 1:1, 1:3, 1:5, 1:8, 1:10, 1:12, 1: 15, 1:20, 1:25, or 1:30.

In one of the specific embodiments, the copolymerization reaction refers to a polymerization reaction of the monomer A and the monomer B performed at 30-60 °C (reaction temperature) under the initiation of an initiator. Specifically, the reaction temperature may be the following specific temperature value: 30 °C, 35 °C, 36 °C, 37 °C, 38 °C, 40 °C, 41 °C, 42 °C, 43 °C, 45 °C, 50 °C, 52 °C, 54 °C, 55 °C, 56 °C, 58 °C, or 60 °C.

In one of the specific embodiments, the initiator is selected from at least one of azobisisobutyronitrile (AIBN) and potassium persulfate (KPS).

In one of the specific embodiments, the polymerization reaction is stopped by oxygen.

In one of the specific embodiments, the polymer is prepared by extracting the product of the polymerization reaction with methanol and drying the product.

The embodiments of the present disclosure further provide a chip including a substrate and a polymer grafted to a surface of the substrate. The polymer is a polymer as described above.

In one of the specific embodiments, the substrate is modified with at least an active group, and the polymer is grafted to the surface of the substrate through the active group. The active group is selected from at least one of amino, epoxy, alkynyl, cyano, vinyl, and propenyl.

In one of the specific embodiments, the biochip further includes a biological component grafted to the polymer. As such, the biochip is used for further biological reactions and applications. Specifically, the biological component is selected from at least one of an amino acid sequence and a nucleotide sequence. Further, the amino acid sequence contains at least one of protein, oligopeptide, polypeptide, or the like; and the nucleotide sequence contains at least one of oligonucleotide sequence, polynucleotide sequence, or the like.

The embodiments of the present disclosure further provide a preparation method for preparing a chip, which includes: obtaining a substrate, and grafting a polymer to a surface of the substrate. The polymer is selected from any one of the above examples. The preparation method for the chip does not need strict control of reaction conditions, and has a simple and easily-controlled process, which is beneficial to popularization and application of the chip.

In one of the specific embodiments, the active group is grafted to the surface of the substrate, then the polymer is grafted to the surface of the substrate through the active group. The active group is selected from at least one of amino, epoxy, alkynyl, cyano, vinyl, and propenyl.

In one of the specific embodiments, grafting the active group refers to subjecting the active group to a reaction with the substrate with a silane coupling agent. The silane coupling agent is selected from at least one of 3-aminopropyltrimethoxysilane, 2-propynyl[3-(triethoxysilyl)propyl]carbamate, 4-(triethoxy)siliylbutyronitrile, and γ-(2,3-epoxypropoxy)propyltrimethoxysilane.

In one of the specific embodiments, grafting of the polymer to the surface of the substrate through the active group refers to a reaction performed at 40-50 °C for 1-8 h (reaction temperature^{∗}time). In particular, the reaction temperature*time may be the following combination: 40 °C*4 h, 43 °C*4 h, 44 °C*4 h, 45 °C*4 h, 46 °C*4 h, 47 °C*4 h, 50 °C*4 h, 45 °C*2 h, 45 °C*6 h, 45 °C^{∗}1 h, or 45 °C*8 h.

In one of the specific embodiments, grafting the active group refers to subjecting the silane coupling agent to a reaction with the substrate at 20-30 °C for 1-5 h.

In addition, in one of the specific embodiments, the preparation method may further include the following step: grafting a biological component to the high polymer. As such, the biochip is used for further biological reactions and applications. Specifically, the biological component is selected from at least one of an amino acid sequence and a nucleotide sequence. Further, the amino acid sequence contains a protein, an oligopeptide, a polypeptide, or the like; and the nucleotide sequence contains an oligonucleotide sequence, a polynucleotide sequence, or the like.

In one of the specific embodiments, grafting the biological component to the high polymer refers to a reaction performed at 50-60 °C for 0.5-5 h (reaction temperature*time). Specifically, the reaction temperature*time may be the following combination: 55 °C*0.5 h, 55 °C^{∗}1 h, 55 °C*2 h, 55 °C*3 h, 55 °C*5 h, 54 °C^{∗}1 h, 56 °C^{∗}1 h, 52 °C^{∗}1 h, 50 °C^{∗}1 h, 58 °C^{∗}1 h, or 60 °C^{∗}1 h.

In addition, the examples of the present disclosure provide use of the polymer as described above, the chip as described above, or a chip prepared by the method as described above in the preparation or analysis of a biomolecule.

The following are specific examples. Unless otherwise specified, the starting materials are all commercially available, and the proportions are all represented by percentages by mass.

T20: tttttttttttttttttttttttttt (SEQ ID NO: 1); Pe: caacaacaacaacaacaacaacaacaa (SEQ ID NO: 2); RD: ctgccccgggttcctcattc tat (SEQ ID NO: 3).

### Example 1

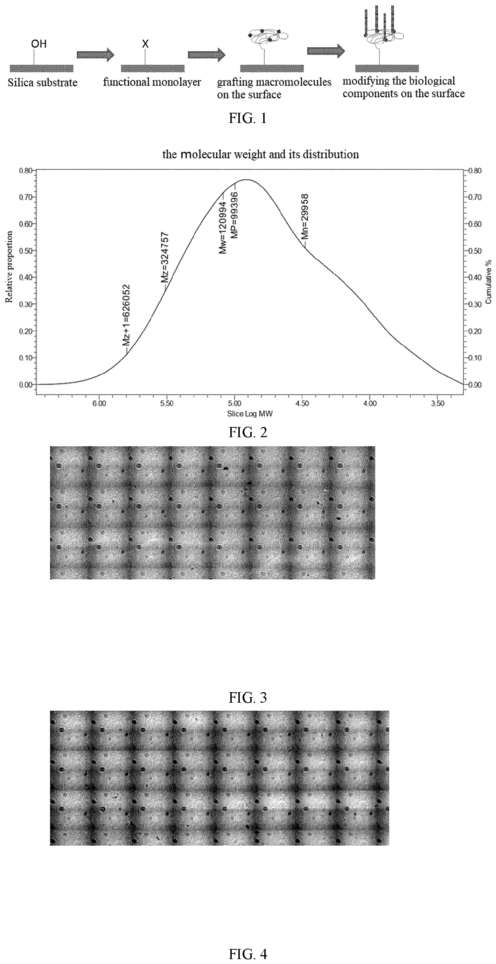

Please refer to FIG. 1.

### (1) Polymer preparation

With toluene as a solvent, according to the volume ratio, 5 wt% GMA (glycidyl methacrylate), 0.1 wt% PEGDA (polyethylene glycol (glycol) diacrylate) with the molecular weight of 1000 g/mol, and 0.1 wt% AIBN (azodiisobutyronitrile or azobisisobutyronitrile) initiator were added to the system, and after nitrogen was charged and oxygen was removed, the polymerization reaction was performed at 55 °C. After the reaction was performed for 3 h, oxygen was injected to stop the reaction. The polymer was extracted with methanol, and dried in vacuum to obtain a stereoscopic epoxy macromolecule, which was detected by GPC (gel permeation chromatography). The detection result is shown in FIG. 2, which shows that an epoxy macromolecule having a peak molecular weight of 98,215 is obtained.

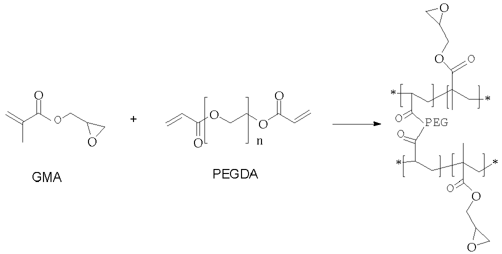

### (2) Substrate surface modification

With a piece of glass as the substrate, the piece of glass was subjected to amination film plating on the surface by a solution method. The film plating method is as follows:
The piece of glass was placed in a solution of 5% APTMS (aminopropyltrimethoxysilane) in ethanol for a reaction at 25 °C for 2 h. The piece of glass was taken out of the solution and baked in an oven at 130 °C for 2 h for the amination on the surface of the piece of glass.

The piece of glass after amination film plating was placed in a solution of 0.1 wt% epoxy macromolecules in isopropanol for a reaction at 45 °C for 4 h to obtain the surface grafted with the epoxy macromolecules.

The surface of the piece of glass modified with the epoxy macromolecules was tested for quality by using NH₂-CY3 (purchased from Xi'an Kaixin Biotechnology Co., Ltd). The specific procedures are as follows:
20 µM NH₂-CY3 solution was charged to the surface for a reaction at 55 °C for 1 h. The light density was detected by photographing under a fluorescence microscope with a laser in the 532 nm band according to the parameters of 35 mW, 60 ms. The light intensity detection result is shown in FIG. 3, and the average surface brightness obtained by the signal statistics using ImageJ is 21,919 a.u.

(3) Oligonucleotide ligation (a) A micro-channel was formed on the surface of the substrate modified with macromolecules by the micro-channel packaging technology, such as surface modification packaging, thermocompressive bonding packaging or anodic bonding micro-channel packaging technology. 5 µM NH₂-oligo (NH₂-A₃₀) solution was added to the micro-channel for a reaction at 37 °C for 24 h to graft a high-density oligo (oligonucleotide) sequence to the surface. A₃₀ indicates an oligonucleotide strand formed by 30 adenine nucleotides.
(b) After the surface of the micro-channel was washed with pure water, 5 µM T₂₀-CY3 solution was added for hybridization at 55 °C for 30 min.
(c) The light density was detected by photographing under a fluorescence microscope with a laser in the 532 nm band according to the parameters of 35 mW, 60 ms. The light intensity detection result is shown in FIG. 4, and the average brightness value obtained is 28,267 a.u.

### Example 2

### (1) Polymer preparation

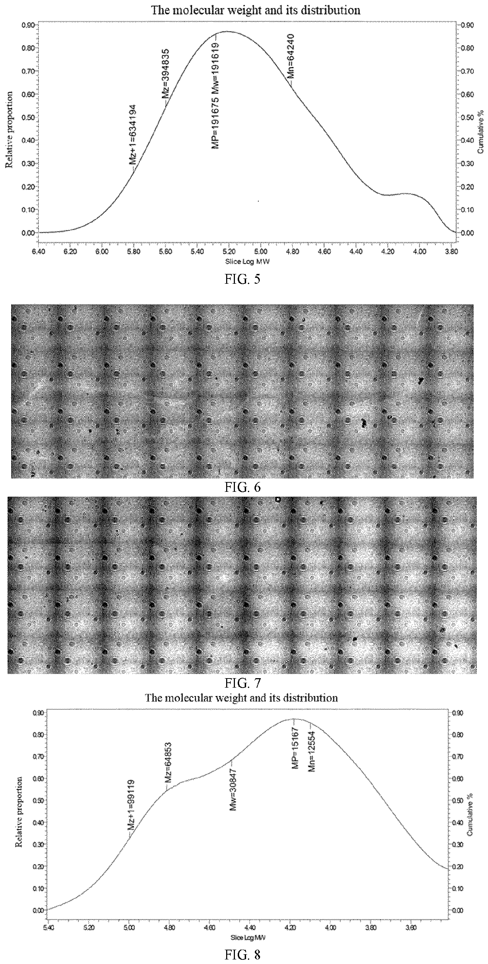

Water and DMF (dimethylformamide) were used as solvents, wherein the volume ratio of water to DMF was 4:1. According to the mass ratio, 2% acrylamide (AM), 0.2% GMA monomer, 0.1% KPS (potassium persulfate) initiator, and 0.01% TEMED (tetramethylethylenediamine) gel accelerant were added to the system, and after nitrogen was charged and oxygen was removed, a polymerization reaction was performed at 42 °C. After the reaction was performed for 3 h, oxygen was injected to stop the reaction. The polymer was extracted with methanol, and dried in vacuum to obtain a linear epoxy macromolecule, which was detected by GPC (gel permeation chromatography). The detection result is shown in FIG. 5, which shows that an epoxy macromolecule having a peak molecular weight of 191,619 was obtained.

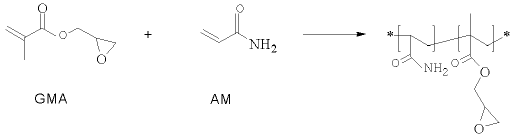

### (2) Substrate surface modification

With a piece of glass as the substrate, the piece of glass was subjected to amination film plating on the surface by a solution method. The film plating method is as follows:
The piece of glass was placed in a solution of 5% APTMS (aminopropyltrimethoxysilane) in ethanol for a reaction at 25 °C for 2 h.
The piece of glass was taken out of the solution and baked in an oven at 130 °C for 2 h for the amination on the surface of the piece of glass.
The piece of glass after amination film plating was placed in a solution of 0.1 wt% epoxy macromolecules in isopropanol for a reaction at 45 °C for 4 h to obtain the surface modified with the epoxy macromolecules.

The surface of the piece of glass modified with the epoxy macromolecules was tested for quality by using NH₂-CY3 (purchased from Xi'an Kaixin Biotechnology Co., Ltd). The specific procedures are as follows:
20 µM NH₂-CY3 solution was charged to the surface for a reaction at 55 °C for 1 h. The light density was detected by photographing with a fluorescence microscope using a laser in the 532 nm band according to the parameters of 35 mW, 60 ms. The light intensity detection result is shown in FIG. 6, and the **average** signal intensity obtained by analysis using ImageJ software is 13,768 a.u.

### (3) Oligonucleotide ligation

(a) A micro-channel was formed on the surface of the substrate modified with macromolecules by the micro-channel packaging technology. 5 µM NH₂-oligo (NH₂-T₃₅) solution was added to the micro-channel for a reaction at 37 °C for 24 h to graft a high-density oligo (oligonucleotide) sequence on the surface. T₃₅ indicates an oligonucleotide strand formed by 35 thymine nucleotides.
(b) After the surface of the micro-channel was washed with pure water, 5 µM A₃₀-CY3 solution was added for hybridization at 55 °C for 30 min. A₃₀ indicates an oligonucleotide strand formed by 30 adenine nucleotides.
(c) The light density was detected by photographing under a fluorescence microscope with a laser in the 532 nm band according to the parameters of 35 mW, 60 ms. The light intensity image is shown in FIG. 7, and the average signal intensity obtained is 13,039 a.u.

### Example 3

### (1) Polymer preparation

Water and DMF (dimethylformamide) were used as solvents, wherein the volume ratio of water to DMF was 4:1. According to the mass ratio, 2% acrylamide (AM), 1% alkenyl azide monomer (purchased from Xi'an Kaixin Biotechnology Co., Ltd), 0.1% KPS initiator, and 0.01% TEMED gel accelerant were added to the system, and after nitrogen was charged and oxygen was removed, a polymerization reaction was performed at 37 °C. After the reaction was performed for 3 h, oxygen was injected to stop the reaction. The polymer was extracted with ethanol, and dried in vacuum to obtain a linear azido macromolecule, which was detected by GPC (gel permeation chromatography). The detection result is shown in FIG. 8, which shows that an azido macromolecule having a peak molecular weight of 15,167 was obtained.

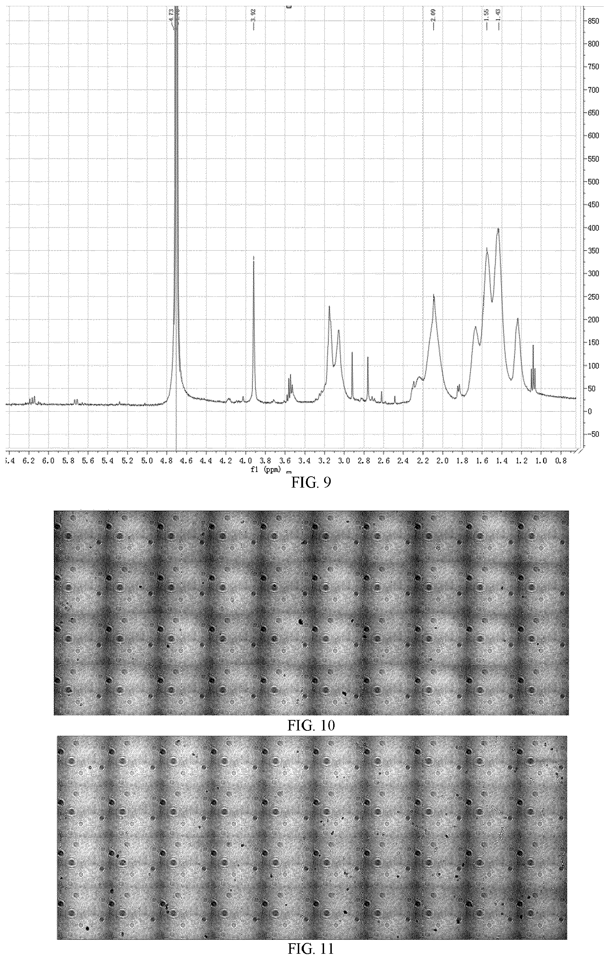

The nuclear magnetic resonance test is shown in FIG. 9, which shows that H absorption peaks of a saturated chain segment formed after olefin polymerization appear at 1.43, 1.55, and 2.09, H absorption peaks of CH₂ in diamine in molecules appear in the range of 3.04-3.41, and H absorption peaks of a PEG chain segment adjacent to the azide appear near 3.93, demonstrating that the azido macromolecule is formed.

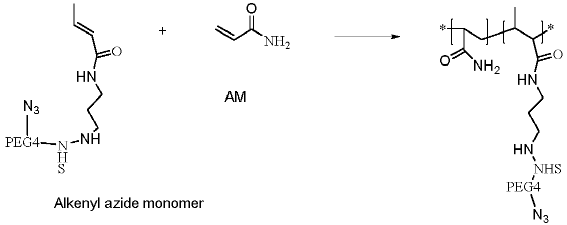

### (2) Substrate surface modification

With a piece of glass as the substrate, the piece of glass was subjected to alkynylation film plating on the surface by a solution method. The film plating method is as follows:

The piece of glass was placed in a 1% toluene solution of 2-propynyl[3-(triethoxysilyl)propyl]carbamate for a reaction at 25 °C for 2 h. The piece of glass was taken out of the solution and baked in an oven at 130 °C for 2 h for the alkynylation on the surface of the piece of glass.

The piece of glass after alkynylation film plating was placed in an isopropanol solution of 0.1 wt% azido macromolecules for a reaction at 45 °C for 4 h to obtain the surface grafted with the azido macromolecules.

The surface of the piece of glass surface modified with the azido macromolecules was tested for quality by using DBCO (dibenzocyclooctyne)-CY3 (purchased from Xi'an Kaixin Biotechnology Co., Ltd). The specific procedures are as follows:
20 µM DBCO-CY3 solution was charged to the surface for a reaction at 55 °C for 1 h. The light density was detected by photographing under a fluorescence microscope with a laser irradiation in the 532 nm band according to the parameters of 35 mW, 60 ms. The light intensity detection result is shown in FIG. 10 and analyzed in ImageJ software to obtain a surface with an average signal intensity of 19,464 a.u.

### (3) Oligonucleotide ligation

(a) A micro-channel was formed on the surface of the substrate modified with macromolecules by the micro-channel packaging technology. 5 µM DBCO-oligo (DBCO-T₃₅) solution was added to the micro-channel for a reaction at 37 °C for 24 h to graft a high-density oligo (oligonucleotide) sequence to the surface. T₃₅ indicates 35 thymine nucleotides.
(b) After the surface was washed with pure water, 5 µM A₃₀-cy3 was added for hybridization at 55 °C for 30 min. A₃₀ indicates an oligonucleotide strand formed by 30 adenine nucleotides.
(c) The light density was detected by photographing under a fluorescence microscope with a laser in the 532 nm band according to the parameters of 35 mW, 60 ms. The light intensity detection result is shown in FIG. 11, and the average signal intensity obtained is 14,535 a.u.

### Example 4

### (1) Polymer preparation

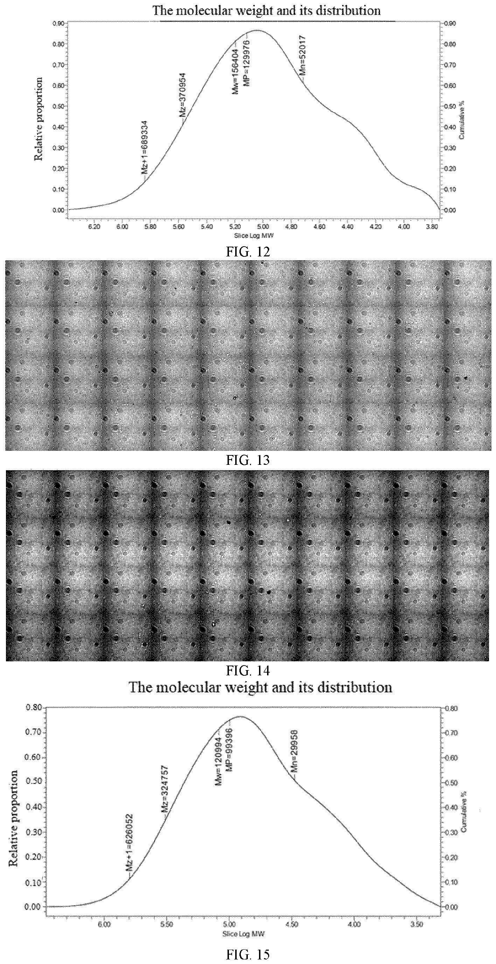

With water as a solvent, according to the mass ratio, 2% alkenyl azide monomer, 0.1 % PEGDA monomer with a molecular weight of 1000 g/mol or 575 g/mol, 0.1% KPS initiator, and 0.01% TEMED gel accelerant were added to the system, and after nitrogen was charged and oxygen was removed, a polymerization reaction was performed at 37 °C. After the reaction was performed for 3 h, the reaction was terminated. The polymer was extracted with ethanol, and dried in vacuum to obtain a stereoscopic azido macromolecule, which was detected by GPC . The detection result is shown in FIG. 12, which shows that an azido macromolecule having a peak molecular weight of 1,299,767 was obtained.

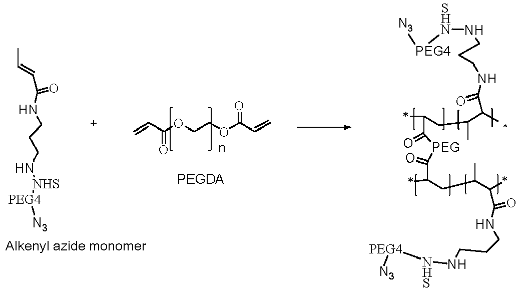

### (2) Substrate surface modification

With a piece of glass as the substrate, the piece of glass was subjected to cyanation film plating on the surface by a solution method. The film plating method is as follows:
The piece of glass was placed in a solution of 1% 4-(triethoxy)siliylbutyronitrile in toluene for a reaction at 25 °C 2 h. The piece of glass was taken out of the solution and baked in an oven at 130 °C for 2 h for the cyanation on the surface of the piece of glass.
The piece of glass after cyanation film plating was placed in a solution of 0.1 wt% azido macromolecules in isopropanol for a reaction at 45 °C for 4 h to obtain the surface grafted with the azido macromolecules. The surface of the piece of glass modified with the azido macromolecules was tested for quality by using DBCO-CY3 (purchased from Xi'an Kaixin Biotechnology Co., Ltd). The specific procedures are as follows:
   20 µM DBCO-CY3 solution was charged to the surface for a reaction at 55 °C for 1 h. The light density was detected by photographing under a fluorescence microscope with a laser in the 532 nm band according to the parameters of 35 mW, 60 ms. The light intensity detection result is shown in FIG. 13, and the average signal intensity obtained by analysis using ImageJ software is 28,531 a.u.

### (3) Oligonucleotide ligation

(a) A micro-channel was formed on the surface of the substrate modified with macromolecules by the micro-channel packaging technology. 5 µM DBCO-oligo (DBCO-T₃₅) solution was added to the micro-channel for a reaction at 37 °C for 24 h to graft a high-density oligo (oligonucleotide) sequence to the surface. T₃₅ indicates an oligonucleotide strand formed by 35 thymine nucleotides.
(b) After the surface was washed with pure water, 5 µM A₃₀-CY3 solution was added for hybridization at 55 °C for 30 min.
(c) The light density was detected by photographing under a fluorescence microscope with a laser in the 532 nm band according to the parameters of 35 mW, 60 ms. The light intensity detection result is shown in FIG. 14, and the average signal intensity obtained is 32,108 a.u.

### Example 5

### (1) Polymer preparation

With water as a solvent, according to the volume ratio, 5% *N-*(3-aminopropyl)methacrylate hydrochloride monomer, 0.1% PEGDA monomer with a molecular weight of 1000 g/mol or 575 g/mol, and 0.1% AIBN initiator were added to the system, and after nitrogen was charged and oxygen was removed, a polymerization reaction was performed at 55 °C. After the reaction was performed for 3 h, oxygen was injected to stop the reaction. The polymer was extracted with methanol, and dried in vacuum to obtain a stereoscopic amino macromolecule, which was detected by GPC (gel permeation chromatography). The detection result is shown in FIG. 15, which shows that an amino macromolecule having a peak molecular weight of 99,396 is obtained.

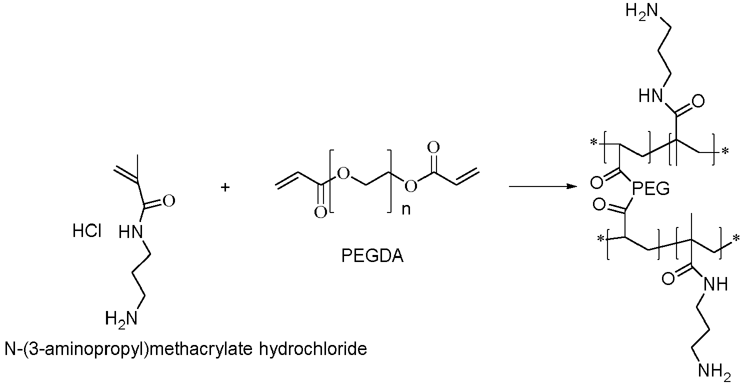

### (2) Substrate surface modification

With a piece of glass as the substrate, the piece of glass was subjected to epoxidation film plating on the surface by a solution method. The film plating method is as follows:
The piece of glass was placed in a solution of 1% γ-(2,3-epoxypropoxy)propyltrimethoxysilane in toluene for a reaction at 25 °C for 2 h. The piece of glass was taken out of the solution and baked in an oven at 130 °C for 2 h for the epoxidation on the surface of the piece of glass.
The piece of glass after epoxidation film plating was placed in a solution of 0.1 wt% amino macromolecules in isopropanol for a reaction at 45 °C for 4 h to obtain the surface grafted with the azido macromolecules.

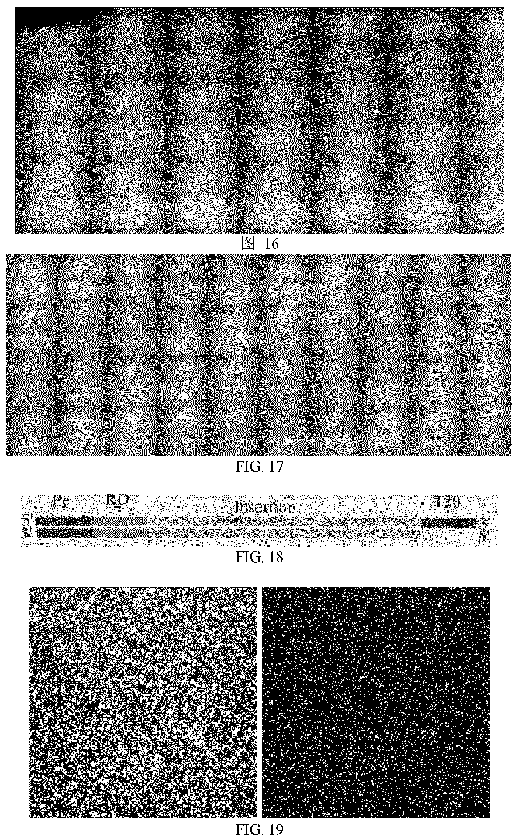

The surface of the piece of glass modified with the azido macromolecules was tested for quality by using NHS-CY3 (customized/purchased from Xi'an Kaixin Biotechnology Co., Ltd). The specific procedures are as follows:
20 µM NHS-CY3 solution was charged to the surface for a reaction at 55 °C for 1 h. The light density was detected by photographing under a fluorescence microscope with a laser irradiation in the 532 nm band according to the parameters of 35 mW, 60 ms. The light intensity detection result is shown in FIG. 16, and the average signal intensity obtained by analysis in ImageJ software is 25,437 a.u.

### (3) Biological application of the prepared biochip:

(a) The piece of glass modified with macromolecules was packaged into a chip (flow cell) which is provided with a micro-channel and can contain a certain amount of liquid. 5 µM NHS-oligo (NHS-T35) solution was added to the micro-channel of the packaged chip for a reaction at 37 °C for 24 h to ensure that the surface was grafted with the high-density primer
(b) After the surface was washed with pure water, 5 µM A30-CY3 solution was added for hybridization at 55 °C for 30 min.
(c) The light density was detected by photographing under a fluorescence microscope with a laser in the 532 nm band according to the parameters of 35 mW, 60 ms. The light intensity detection result is shown in FIG. 17, and the average signal intensity obtained is 33,155 a.u.

### Example 6

Library hybridization and detection were performed using the chips prepared in Examples 1-5. The specific procedures are as follows:

### 1. Preparation of DNA hybridization library

DNA library: DNA library of fragments with a length of 150-300 bp and known sequences at both ends, the molecular structure of the library is shown in FIG. 18; Insertion: an inserted fragment derived from phi-X174 standard strain; and T20, Pe and RD are respectively sequences set forth in SEQ ID NOs: 1-3.

The DNA library was mixed with 52 µL of deionized water, and 18 µL of 0.2 M NaOH solution was added. After uniformly mixing, the mixture was left to stand for denaturation at room temperature for 8 min, and then the reaction was stopped by adding 20 µL of 400 mM Tris-HCl buffer, pH 8.0 to obtain 100 µL of 100 pM denatured DNA library.

### 2. Hybridization of denatured DNA library with chip probes

The denatured DNA library was diluted to 5 pM using a hybridization solution containing 3× SSC (20× SSC buffer diluted with RNase-free water), pH 7.3. The diluted DNA library was charged into the channel of the chip and the chip was incubated for a hybridization reaction at 42 °C for 30 min. 160-260 µL of washing reagent (5× SSC, 0.05% Tween 20, pH 7.0) was charged at a rate of 250 µL/min to complete the hybridization reaction.

### 3. Initial extension of the template

1) 160 to 260 µL of extension buffer reagent (20 mM tris(hydroxymethyl)aminomethane (Tris), 10 mM ammonium sulfate, 2 mM magnesium sulfate, 1.5 M betaine, 1.3% dimethyl sulfoxide, 0.45 M *N*-methyl formamide, 1.5 M carboxamide, and 0.1% TritonX-100, pH 9.0) was charged into the channel of the chip at a rate of 500 µL/min;
2) 160-260 µL of extension reagent (extension buffer reagent, 3 µg/mL Bst DNA polymerase, 200 µM dNTPs) was charged into the channel of the chip at a rate of 500 µL/min, and the chip was incubated for a reaction at 50-60 °C for 5 min to complete initial extension of the template.

### 4. DNA cluster generation

The amplification for clustering may be performed using the ILLUMINA sequencing platform operating manual; the amplification may also be performed using the template walking technique disclosed in the article Isothermal amplification method for next-generation sequencing (ZHAOchun Ma, et al., PNAS August 27, 2013 110 (35) 14320-14323, https://doi.org/10.1073/pnas.1311334110) to generate DNA clusters.

### 5. DNA cluster detection

1) The thermal cycle temperature was set to 50 to 60 °C;
2) 160-260 µL of denaturing reagent formamide was charged into the channel of the chip at a rate of 500 µL_{/}min The chip was denatured for 5 min to break the DNA double-helix structure;
3) 160-260 µL of quality control reagent (0.5 µM RD-Cy3, 3× SSC) was charged into the channel of the chip at a rate of 500 µL/min, where Cy3 in RD-Cy3 was located at the 5' end of RD sequence;
4) The thermal cycle temperature was set to 25 °C and the chip was incubated for a reaction for 15 to 30 min;
5) 160-260 µL of washing reagent was charged into the channel of the chip at a rate of 500 µL/min;
6) Images were taken on a fluorescence detection system using a 20-fold objective lens, with a wavelength of 532 nm, a laser power of 300 mW, and an exposure time of 20 ms.

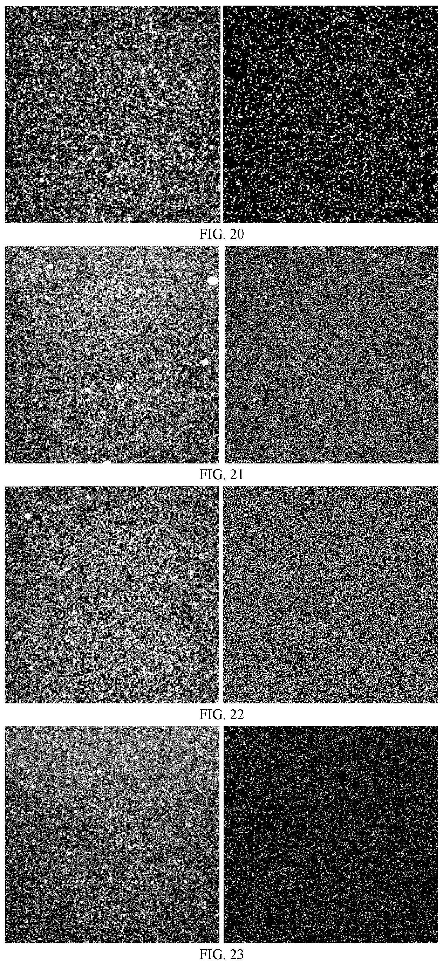

The results of DNA cluster detection are shown in FIG 19-23. and Table 1, and the results of DNA cluster detection corresponding to chips prepared in Examples 1-5 are shown in FIGs. 19-23, respectively.

**Table 1**

| Biochip | Density (cluster/µm²) | Density (cluster/mm²) | Average luminance per cluster (cts) |
|---|---|---|---|
| Example 1 | 0.0692875 | 69288 | 1906 |
| Example 2 | 0.1271716 | 127172 | 1145 |
| Example 3 | 0.1742481 | 174248 | 1730 |
| Example 4 | 0.1519920 | 151992 | 5116 |
| Example 5 | 0.0860361 | 86036 | 399 |

As can be seen from Table 1, the chips prepared in Examples 1-5 can be successfully used for hybridization of the library and DNA cluster generation.

In addition, further, by loading the chip with the surface containing the DNA clusters into a sequencing platform, such as the sequencing platform of ILLUMINA to perform sequencing, sequencing data with quality meeting the requirements of specific applications can be obtained.

In the description of this specification, the description of the terms "one embodiment", "certain embodiments", "schematic embodiments", "examples", "specific examples", "some examples", or the like, means that the particular features, structures, materials or characteristics comprised in the embodiments or examples are included in at least one embodiment or example of the present disclosure. In this specification, the schematic description of the aforementioned terms does not necessarily refer to the same embodiment or example. Moreover, the particular features, structures, materials or characteristics described may be combined in any embodiment or example in any appropriate manner.

Although the embodiments of the present application have been shown and described above, it can be understood that the aforementioned embodiments are exemplary and are not to be construed as limiting the present application, and that those of ordinary skill in the art may make changes and modification to such embodiments, without departing from the scope of the present application.

## Claims

1. A substrate having a surface, wherein the surface comprises a polymer coating covalently linked thereto, wherein the polymer coating comprises a polymer comprising a repeating unit A of formula I and a repeating unit B of formula II or formula III: 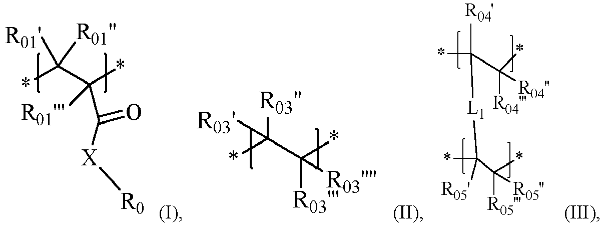 wherein,
X is selected from -O- and -NH-;
R₀₁', R₀₁", and R₀₁‴ are each independently selected from -H and C1-C3 alkyl;
R₀ is selected from C1-C10 alkyl and -(C1-C5 alkyl)-NH-NHS-PEG4, and R₀ comprises at least one R₀₂ substituent, wherein at least one R₀₂ substituent is each independently selected from epoxy, amino, and azido;
R₀₃', R₀₃", R₀₃‴, R₀₃‴′, R₀₄', R₀₄", R₀₄‴, R₀₅', R₀₅", and R₀₅‴ are each independently selected from -H, C1-C3 alkyl, amido, and an ester group;
L₁ is selected from C1-C3 alkylene and -C(O)-R₀₆-C(O)-; and
R₀₆ is selected from PEG and alkyl diamine, and in the case that R₀₆ is selected from PEG, the PEG has a molecular weight of 200-2000.

2. The substrate according to claim 1, wherein the polymer comprises a structure of formula III, formula IV, or formula V:

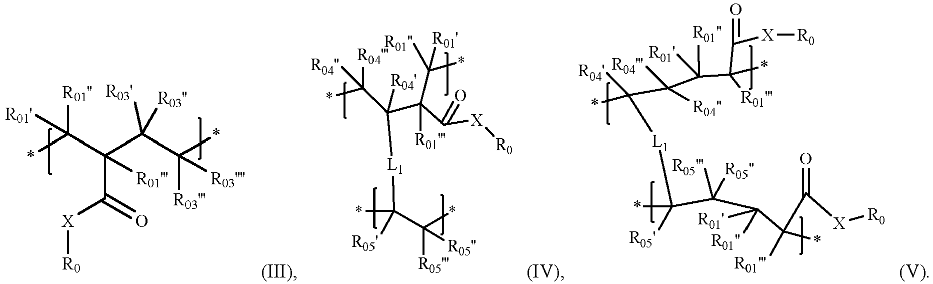

3. The substrate according to claim 1 or 2, wherein the polymer coating comprises a polymer of formula VI or formula VII: 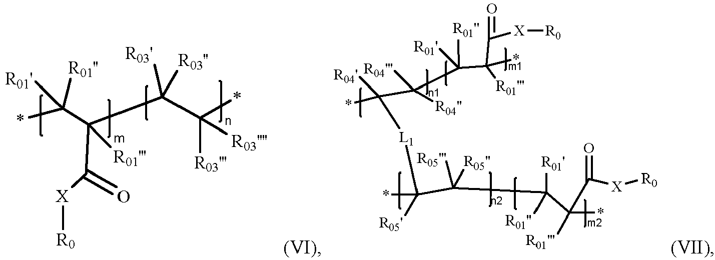 wherein,
m is selected from integers in a range of 1-2000,
n is selected from integers in a range of 1-3000,
m1 and m2 are each independently selected from integers in a range of 1-2000, and
n1 or n2 is selected from integers in a range of 1-1500 and n1 is equal to n2, and in the case that R₀₆ is selected from PEG, the PEG has a molecular weight of 200-2000.

4. The substrate according to claim 3, wherein the polymer coating comprises the polymer of formula VI, wherein a ratio of m to n is 1:1-1:30, preferably 1:2-1:30, and more preferably 1:10-1:20.

5. The substrate according to claim 3, wherein the polymer coating comprises the polymer of formula VII, wherein a ratio of the sum of m1 and m2 to n1 or n2 is 1:1-1:30, preferably 1:2-1:30, and more preferably, 1:10-1:20.

6. The substrate according to any one of claims 1-5, wherein the polymer has a molecular weight of 1-200,000.

7. The substrate according to any one of claims 1-6, wherein the polymer coating is linked to the surface by covalent binding to an active group on the surface, wherein the active group is selected from at least one of amino, epoxy, alkynyl, cyano, vinyl, and propenyl.

8. The substrate according to claim 7, wherein the active group is an amino group, and the polymer comprises an epoxy group.

9. The substrate according to claim 7, wherein the active group is an epoxy group, and the polymer comprises -NH₂.

10. The substrate according to claim 7, wherein the active group is one of alkynyl, cyano, vinyl, and propenyl, and the polymer comprises -N₃.

11. The substrate according to any one of claims 1-10, wherein X is selected from -O-.

12. The substrate according to claim 11, wherein R₀₁', R₀₁", and R₀₁‴ are each independently selected from -H and methyl, and R₀ is selected from C1-C3 alkyl; and/or
R₀₃', R₀₃", R₀₃‴, R₀₃‴′ R₀₄', R₀₄", R₀₄‴, R₀₅', R₀₅", and R₀₅‴ are each independently selected from -H, methyl, -C(O)NH₂, and -C(O)OCH₃, and L₁ is selected from -C(O)-PEG-C(O)- and -C(O)-NH-CH₂-NH-C(O)-.

13. The substrate according to claim 12, wherein the repeating unit A is selected from one of the following structures:

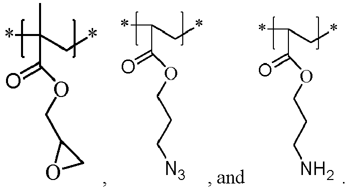

14. The substrate according to any one of claims 11-13, wherein the repeating unit B is selected from one of the following structures: 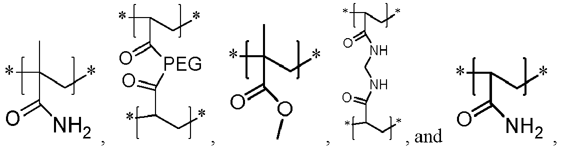 wherein the PEG has a molecular weight of 200-2000, preferably 500-1000.

15. The substrate according to claim 14, wherein the polymer comprises one of the following structures: 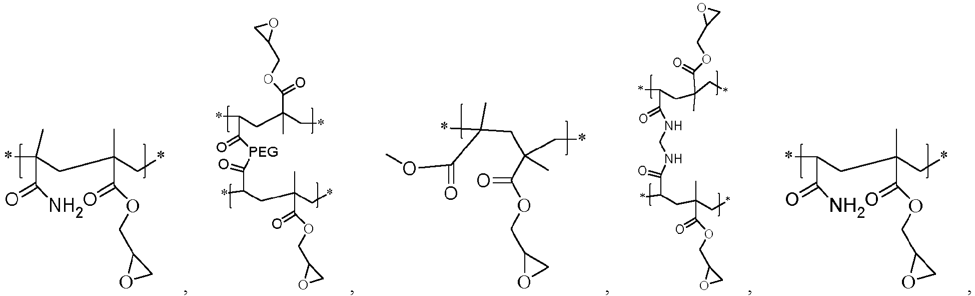 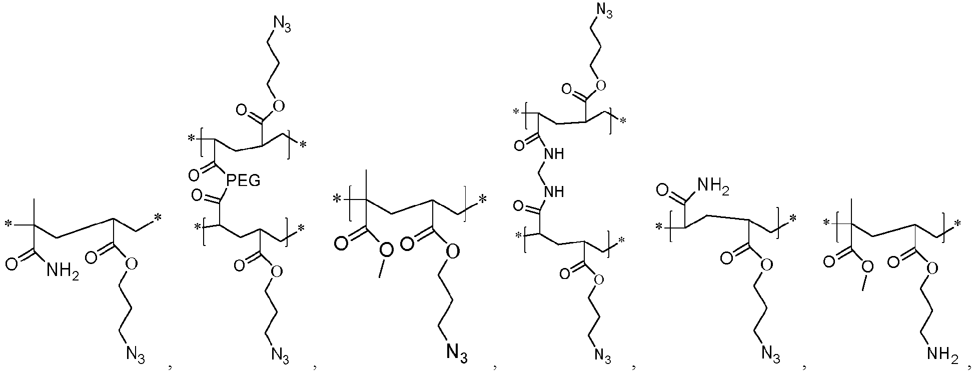 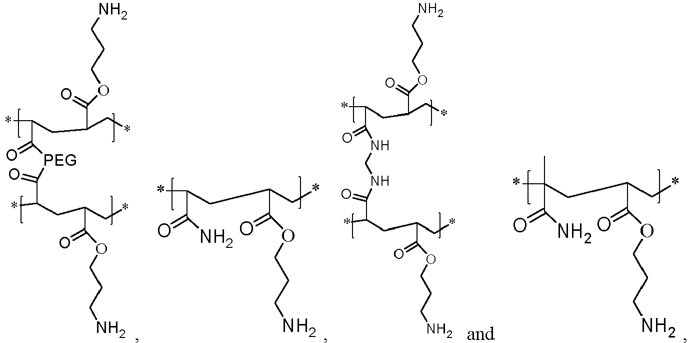 wherein the PEG has a molecular weight of 200-2000, preferably 500-1000.

16. The substrate according to claim 14, wherein the polymer coating comprises one of the following polymers: 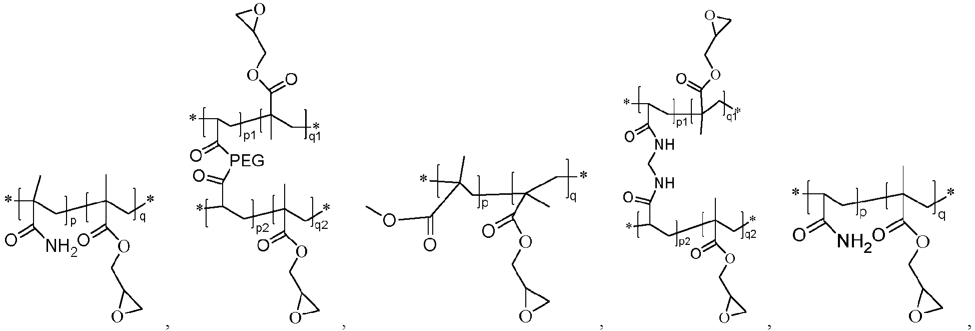 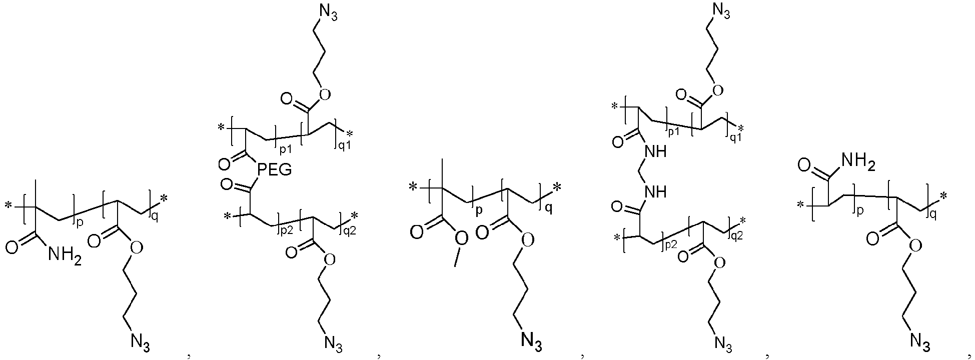 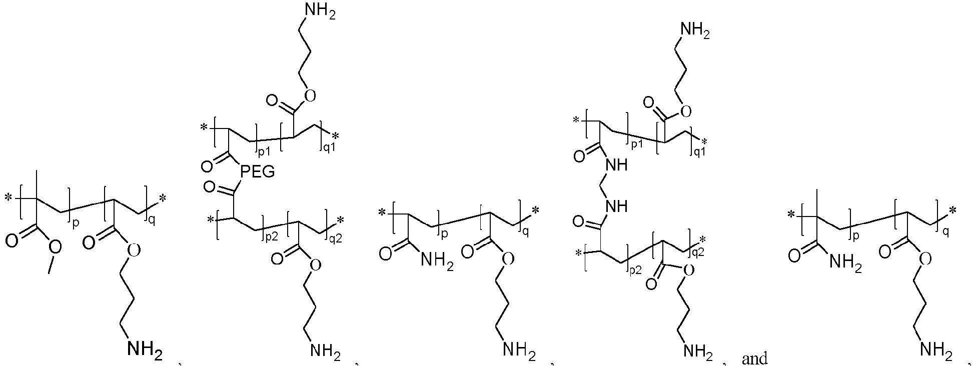 wherein,
p is selected from integers in a range of 1-3000,
p1 and p2 are each independently selected from integers in a range of 1-1300 and p1 is equal to p2,
q, q1, and q2 are each independently selected from integers in a range of 1-2000, and
the PEG has a molecular weight of 200-2000, preferably 500-1000.

17. The substrate according to any one of claims 1-10, wherein X is selected from -NH-.

18. The substrate according to claim 17, wherein the repeating unit B is as shown in formula III.

19. The substrate according to claim 18, wherein R₀₁', R₀₁", and R₀₁‴ are each independently selected from -H and methyl, and R₀ is selected from C1-C3 alkyl and -(C1-C5 alkyl)-NH-NHS-PEG4; and/or
R₀₄', R₀₄", R₀₄‴, R₀₅', R₀₅", and R₀₅‴ are each independently selected from -H, methyl, -C(O)NH₂, and -C(O)OCH₃, and L₁ is selected from -C(O)-PEG-C(O)- and -C(O)-NH-CH₂-NH-C(O)-.

20. The substrate according to claim 19, wherein the repeating unit A is selected from one of the following structures:

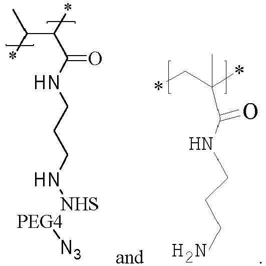

21. The substrate according to any one of claims 18-20, wherein the repeating unit B is selected from one of the following structures: 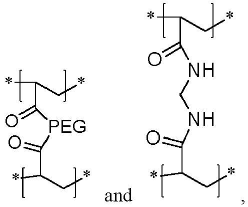 wherein, the PEG has a molecular weight of 200-2000, preferably 500-1000.

22. The substrate according to claim 21, wherein the polymer comprises one of the following structures:

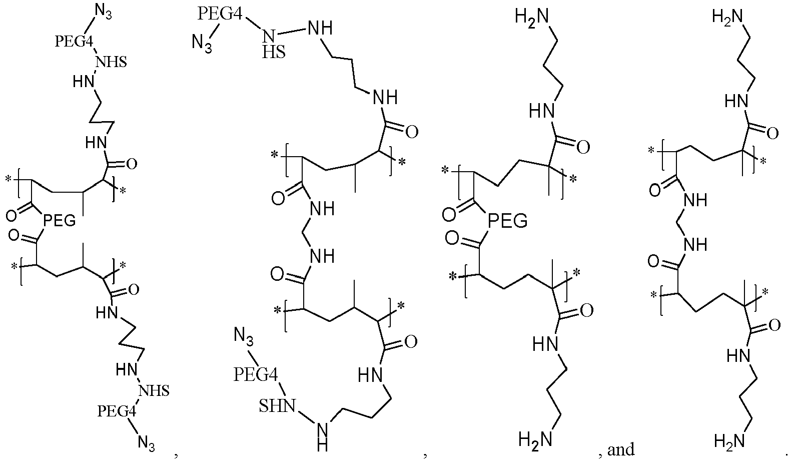

23. The substrate according to claim 21, wherein the polymer coating comprises one of the following polymers: 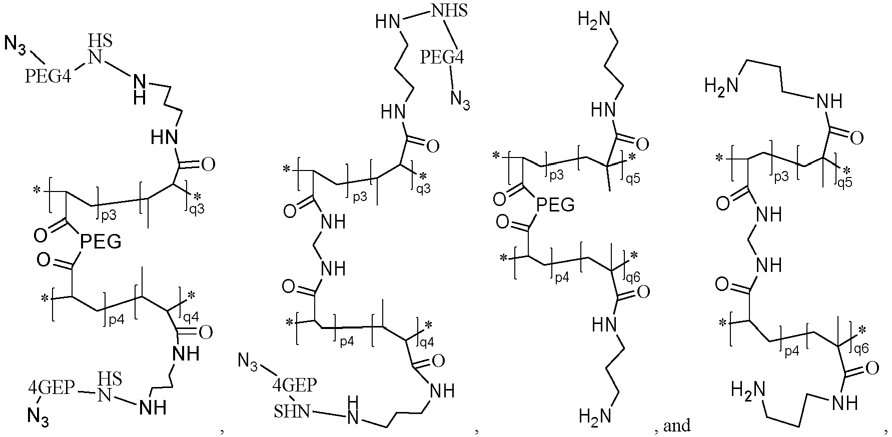 wherein,
p3 and p4 are each independently selected from integers in a range of 1-1300 and p3 is equal to p4,
q3 and q4 are each independently selected from integers in a range of 1-500, and
q5 and q6 are each independently selected from integers in a range of 1-1500.

24. The substrate according to claim 17, wherein the repeating unit B is as shown in formula II.

25. The substrate according to claim 24, wherein the repeating unit B is selected from one of the following structures:

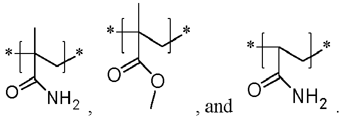

26. The substrate according to claim 25, wherein R₀ is selected from -(C1-C5 alkyl)-NH-NHS-PEG4-N₃.

27. The substrate according to any one of claims 24-26, wherein the repeating unit A is

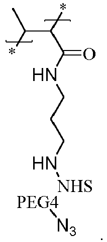

28. The substrate according to claim 27, wherein the polymer comprises one of the following structures:

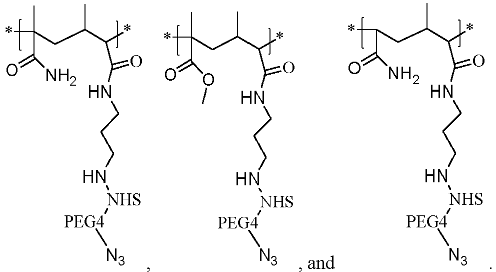

29. The substrate according to claim 27, wherein the polymer coating comprises one of the following polymers: 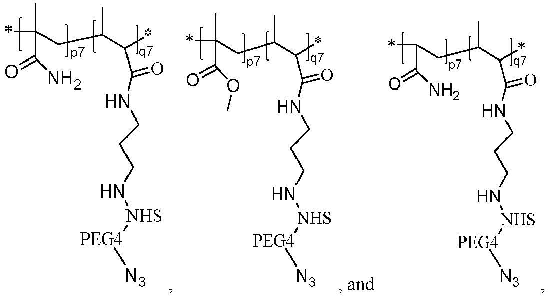 wherein,
p7 is selected from integers in a range of 1-3000, and
q7 is selected from integers in a range of 1-500.

30. The substrate according to claims 1-29, wherein a monomer A of formula VIII corresponding to the repeating unit A and a monomer B of formula IX or formula X corresponding to the repeating unit B are subjected to a polymerization reaction to obtain the polymer, wherein the polymerization reaction is performed in the presence of an initiator, 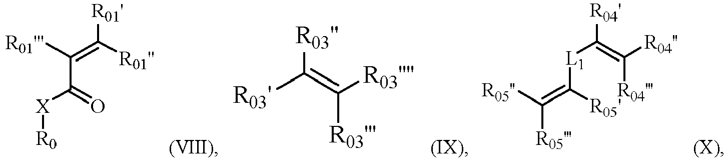 wherein,
X is selected from -O- and -NH-;
R₀₁', R₀₁", and R₀₁‴ are each independently selected from -H and C1-C3 alkyl;
R₀ is selected from C1-C10 alkyl and -(C1-C5 alkyl)-NH-NHS-PEG4, and R₀ comprises at least one R₀₂ substituent, wherein at least one R₀₂ substituent is each independently selected from epoxy, amino, and azido;
R₀₃', R₀₃", R₀₃‴, R₀₃‴′, R₀₄', R₀₄", R₀₄‴, R₀₅', R₀₅", and R₀₅‴ are each independently selected from -H, C1-C3 alkyl, amido, and an ester group;
L₁ is selected from C1-C3 alkylene and -C(O)-R₀₆-C(O)-; and
R₀₆ is selected from PEG and alkyl diamine, and in the case that R₀₆ is selected from PEG, the PEG has a molecular weight of 200-2000, preferably 500-1000.

31. The substrate according to claim 30, wherein a molar ratio of the monomer A to monomer B in the polymerization reaction is selected from a numerical value in a range of 1:1-1:30.

32. The substrate according to any one of claims 1-31, wherein the surface is further linked with a biomolecule, wherein the biomolecule is linked to the surface by grafting to the polymer.

33. The substrate according to claim 32, wherein the biomolecule is selected from at least one of a protein and a nucleic acid.

34. A method for preparing a substrate having a polymer coating on a surface thereof, comprising:
making a polymer in contact with the surface to allow the polymer to be linked to the surface, wherein the polymer comprises a repeating unit A of formula I and a repeating unit B of formula II or formula III: 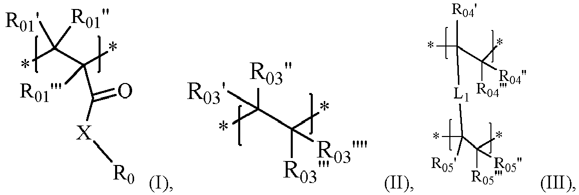 wherein,
X is selected from -O- and -NH-;
R₀₁', R₀₁", and R₀₁‴ are each independently selected from -H and C1-C3 alkyl;
R₀ is selected from C1-C10 alkyl and -(C1-C5 alkyl)-NH-NHS-PEG4, and R₀ comprises at least one R₀₂ substituent, wherein at least one R₀₂ substituent is each independently selected from epoxy, amino, and azido;
R₀₃', R₀₃", R₀₃‴, R₀₃‴′, R₀₄', R₀₄", R₀₄‴, R₀₅', R₀₅", and R₀₅‴ are each independently selected from -H, C1-C3 alkyl, amido, and an ester group;
L₁ is selected from C1-C3 alkylene and -C(O)-R₀₆-C(O)-; and
R₀₆ is selected from PEG and alkyl diamine, and in the case that R₀₆ is selected from PEG, the PEG has a molecular weight of 200-2000.

35. The method according to claim 34, wherein the polymer comprises a structure of formula III, formula IV, or formula V:

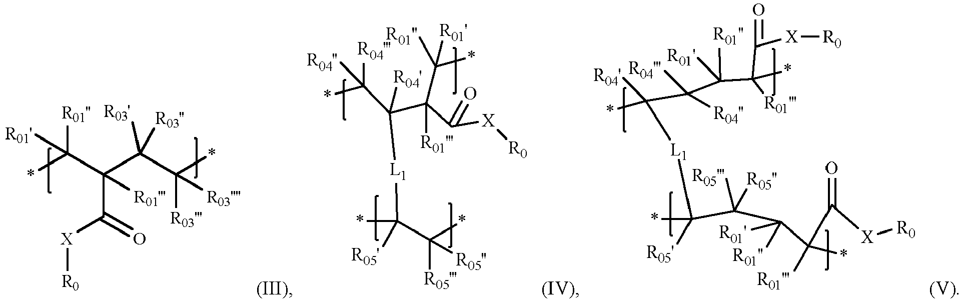

36. The method according to claim 34 or 35, wherein the polymer coating comprises a polymer of formula VI or formula VII: 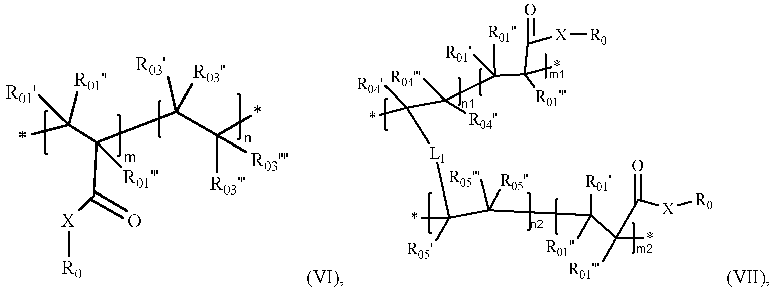 wherein,
m is selected from integers in a range of 1-2000,
n is selected from integers in a range of 1-3000,
m1 and m2 are each independently selected from integers in a range of 1-2000, and
n1 or n2 is selected from integers in a range of 1-1500 and n1 is equal to n2, and in the case that R₀₆ is selected from PEG, the PEG has a molecular weight of 200-2000.

37. The method according to claim 36, wherein the polymer coating comprises the polymer of formula VI, wherein a ratio of m to n is 1:1-1:30, preferably 1:2-1:30, and more preferably 1:10-1:20.

38. The method according to claim 36, wherein the polymer coating comprises the polymer of formula VII, wherein a ratio of the sum of m1 and m2 to n1 or n2 is 1:1-1:30, preferably 1:2-1:30, and more preferably, 1:10-1:20.

39. The method according to any one of claims 34-38, wherein the polymer has a molecular weight of 1-20,0000.

40. The method according to any one of claims 34-39, wherein X is selected from -O-.

41. The method according to claim 40, wherein R₀₁', R₀₁", and R₀₁‴ are each independently selected from -H and methyl, and R₀ is selected from C1-C3 alkyl; and/or
R₀₃', R₀₃", R₀₃‴, R₀₃‴′ R₀₄', R₀₄", R₀₄‴, R₀₅', R₀₅", and R₀₅‴ are each independently selected from -H, methyl, -C(O)NH₂, and -C(O)OCH₃, and L₁ is selected from -C(O)-PEG-C(O)- and -C(O)-NH-CH₂-NH-C(O)-, and in the case that L₁ is selected from -C(O)-PEG-C(O)-, the PEG has a molecular weight of 500-1000.

42. The method according to claim 41, wherein the repeating unit A is selected from one of the following structures:

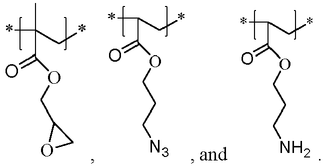

43. The method according to any one of claims 40-42, wherein the repeating unit B is selected from one of the following structures: 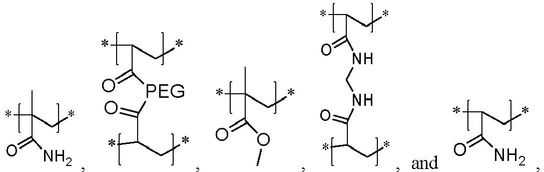 wherein the PEG has a molecular weight of 200-2000, preferably 500-1000.

44. The method according to claim 43, wherein the polymer comprises one of the following structures:

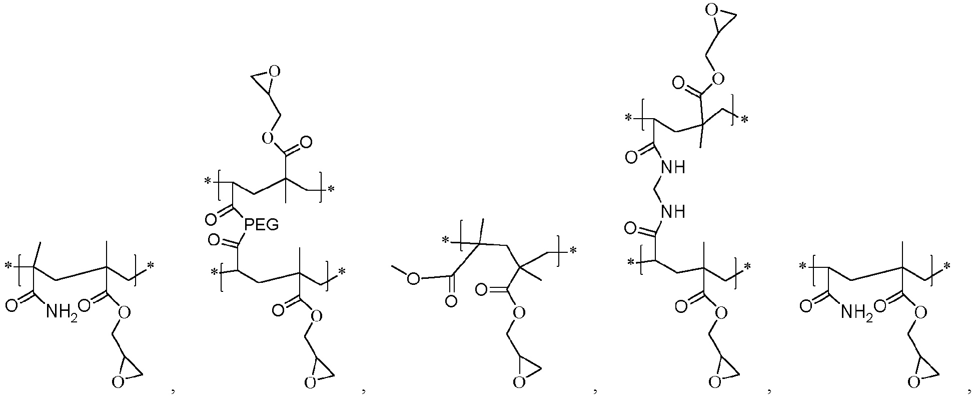

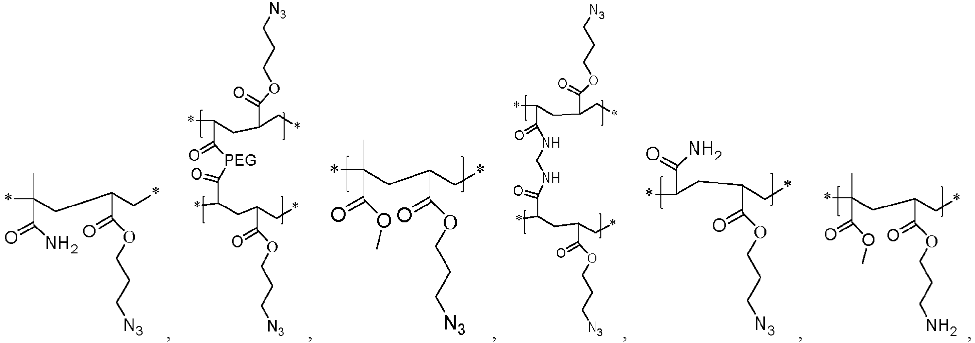

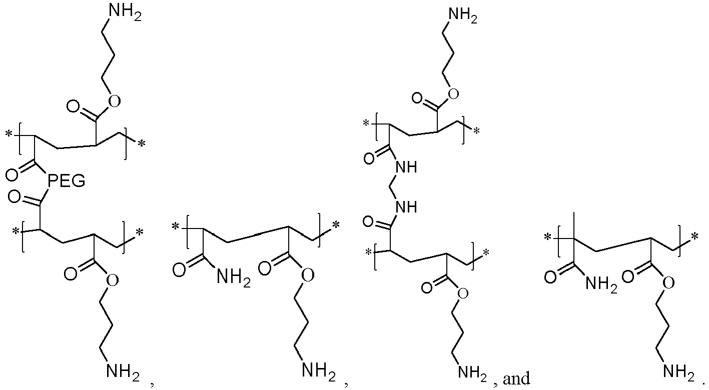

45. The method according to claim 43, wherein the polymer comprises one of the following polymers: 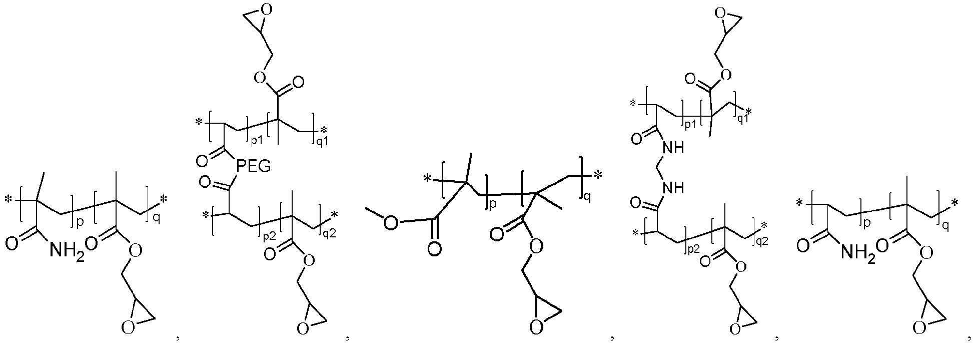 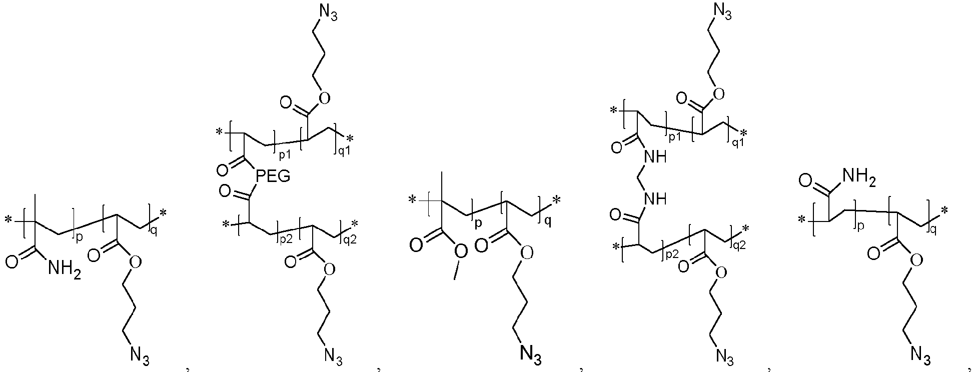 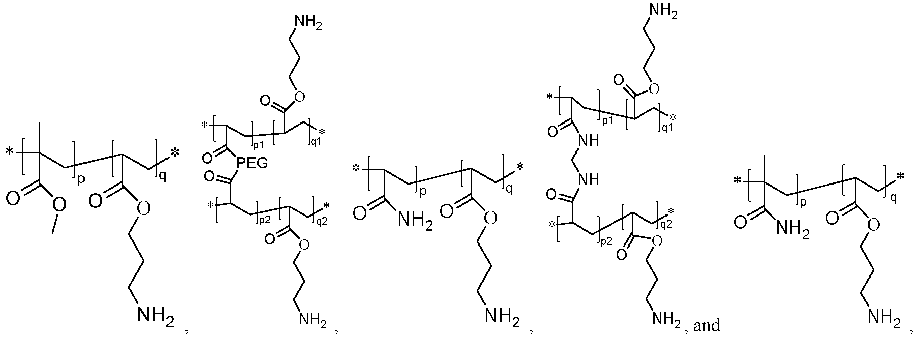 wherein,
p is selected from integers in a range of 1-3000,
p1 and p2 are each independently selected from integers in a range of 1-1300 and p1 is equal to p2, and
q, q1, and q2 are each independently selected from integers in a range of 1-2000.

46. The method according to any one of claims 34-39, wherein X is selected from -NH-.

47. The method according to claim 46, wherein the repeating unit B is as shown in formula III.

48. The method according to claim 47, wherein R₀₁', R₀₁", and R₀₁‴ are each independently selected from -H and methyl, and R₀ is selected from C1-C3 alkyl and -(C1-C5 alkyl)-NH-NHS-PEG4; and/or
R₀₄', R₀₄", R₀₄‴, R₀₅', R₀₅", and R₀₅‴ are each independently selected from -H, methyl, -C(O)NH₂, and -C(O)OCH₃, and L₁ is selected from -C(O)-PEG-C(O)- and -C(O)-NH-CH₂-NH-C(O)-, and in the case that L₁ is selected from -C(O)-PEG-C(O)-, the PEG has molecular weight of 200-2000, preferably 500-1000.

49. The method according to claim 48, wherein the repeating unit A is selected from one of the following structures:

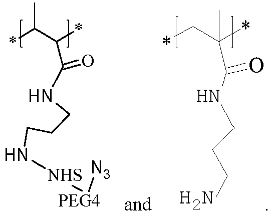

50. The method according to any one of claims 46-49, wherein the repeating unit B is selected from one of the following structures: 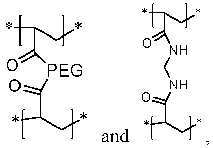 wherein the PEG has a molecular weight of 200-2000, preferably 500-1000.

51. The method according to claim 50, wherein the polymer comprises one of the following structures:

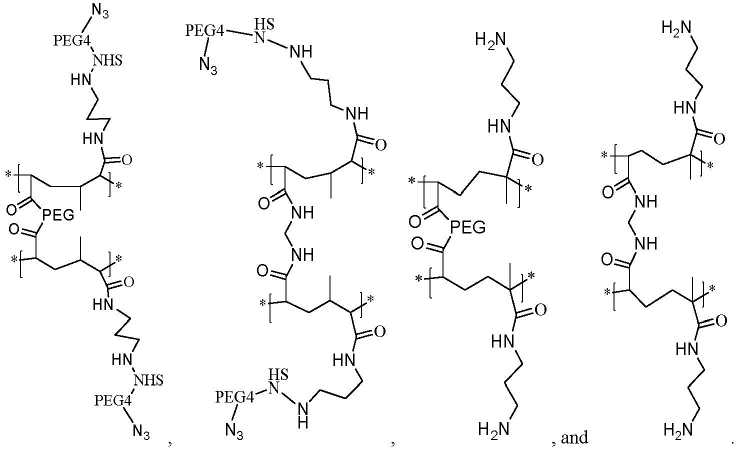

52. The method according to claim 50, wherein the polymer comprises one of the following polymers: 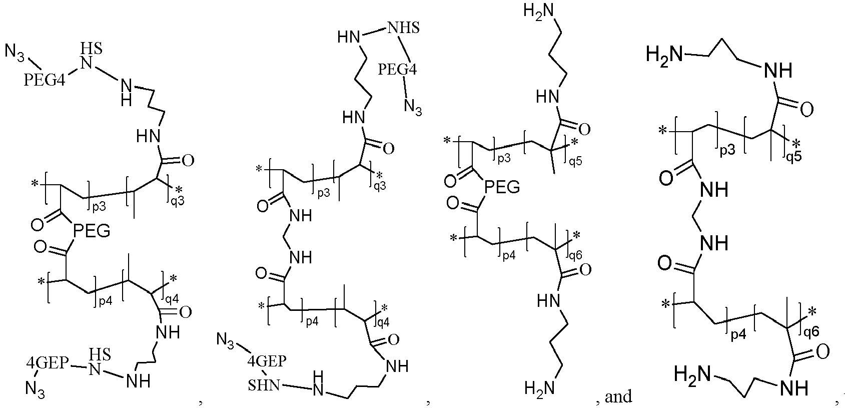 wherein,
p3 and p4 are each independently selected from integers in a range of 1-1300 and p3 is equal to p4,
q3 and q4 are each independently selected from integers in a range of 1-500, and
q5 and q6 are each independently selected from integers in a range of 1-1500.

53. The method according to claim 46, wherein the repeating unit B is as shown in formula II.

54. The method according to claim 53, wherein the repeating unit B is selected from one of the following structures:

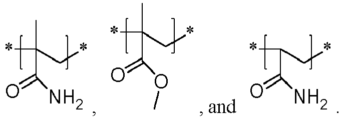

55. The method according to claim 53, wherein R₀ is selected from -(C1-C5 alkyl)-NH-NHS-PEG4-N₃.

56. The method according to any one of claims 53-55, wherein the repeating unit A is

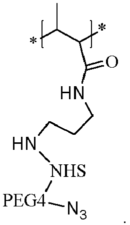

57. The method according to claim 56, wherein the polymer comprises one of the following structures:

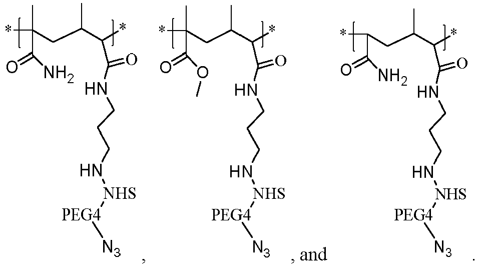

58. The method according to claim 56, wherein the polymer comprises one of the following polymers: 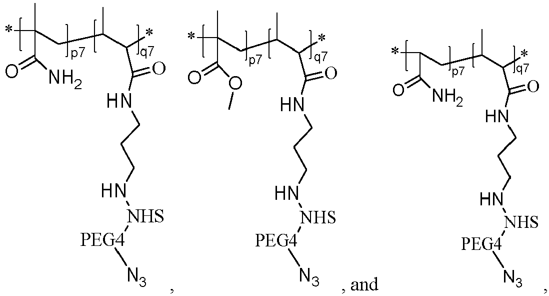 wherein,
p7 is selected from integers in a range of 1-3000, and
q7 is selected from integers in a range of 1-500.

59. The method according to any one of claims 34-58, comprising:
modifying the surface to provide the surface with at least an active group selected from at least one of amino, epoxy, alkynyl, cyano, vinyl, and propenyl; and
making the polymer in contact with the surface to allow the polymer to be linked to the surface by covalently binding to the active group.

60. The method according to claim 59, wherein the active group is an amino group, and the polymer comprises an epoxy group, wherein at least a portion of the epoxy group is subjected to a reaction with at least a portion of the amino group to allow the polymer to be linked to the surface.

61. The method according to claim 59, wherein the active group is an epoxy group, and the polymer comprises an amino group, wherein at least a portion of the amino group is subjected to a reaction with at least a portion of the epoxy group to allow the polymer to be linked to the surface.

62. The method according to claim 59, wherein the active group is selected from one of alkynyl, cyano, vinyl, and propenyl, and the polymer comprises -N₃, and at least a portion of the -N₃ is subjected to a reaction with at least a portion of the active group to allow the polymer to be linked to the surface.

63. The method according to claim 59, wherein the surface is treated with a silane coupling agent to provide the surface with the active group,
wherein the silane coupling agent is selected from at least one of 3-aminopropyltrimethoxysilane, 2-propynyl[3-(triethoxysilyl)propyl]carbamate, 4-(triethoxy)silylbutyronitrile, and γ-(2,3-epoxypropoxy)propyltrimethoxysilane.

64. The method according to claim 63, wherein a reaction is performed at 40-50 °C for 1-8 h to allow the polymer to be linked to the surface by covalently binding to the active group.

65. The method according to any one of claims 34-64, further comprising:
allowing a biomolecule to be linked to the surface by covalently binding to the polymer.

66. The method according to claim 65, wherein the biomolecule is selected from at least one of a protein and a nucleic acid.

67. The method according to claim 66, wherein the a reaction is performed at 50-60 °C for 0.5-5 h to allow the biomolecule to be covalently bound to the polymer.

68. The method according to claim 65, wherein the polymer comprises an epoxy group, and the biomolecule has an amino modification on at least one terminus.

69. The method according to claim 65, wherein the polymer comprises an amino group, and the biomolecule has NHS on at least one terminus.

70. The method according to claim 65, wherein the polymer comprises -N₃, and the biomolecule has DBCO on at least one terminus.

71. Use of the substrate according to any one of claims 1-33 in biomolecule capture and/or detection.
